(19) Europäisches Patentamt
European Patent Office
Office européen des brevets

(11) **EP 4 376 010 B1**

(12) **EUROPEAN PATENT SPECIFICATION**

(45) Date of publication and mention
of the grant of the patent:
**26.11.2025 Bulletin 2025/48**

(21) Application number: **23841899.0**

(22) Date of filing: **23.05.2023**

(51) International Patent Classification (IPC):
**G06V 20/69** (2022.01)   **G06V 20/70** (2022.01)
**G06V 10/82** (2022.01)   **G06V 10/762** (2022.01)
**G06V 10/80** (2022.01)   **G16B 25/10** (2019.01)
**G06T 11/20** (2006.01)

(52) Cooperative Patent Classification (CPC):
**G06T 11/206; G06V 10/762; G06V 10/82;
G06V 20/695; G16B 25/10;** G06V 2201/04

(86) International application number:
**PCT/CN2023/095787**

(87) International publication number:
**WO 2024/016830 (25.01.2024 Gazette 2024/04)**

(54) **VIDEO PROCESSING METHOD AND APPARATUS, DEVICE, AND STORAGE MEDIUM**

VIDEOVERARBEITUNGSVERFAHREN UND -VORRICHTUNG, VORRICHTUNG UND SPEICHERMEDIUM

PROCÉDÉ ET APPAREIL DE TRAITEMENT VIDÉO, DISPOSITIF ET SUPPORT DE STOCKAGE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **22.07.2022 CN 202210871878**

(43) Date of publication of application:
**29.05.2024 Bulletin 2024/22**

(73) Proprietor: **TENCENT TECHNOLOGY
(SHENZHEN) COMPANY LIMITED
Shenzhen, Guangdong 518057 (CN)**

(72) Inventors:
• **WU, Zihan**
**Shenzhen, Guangdong 518057 (CN)**
• **YAO, Jianhua**
**Shenzhen, Guangdong 518057 (CN)**

(74) Representative: **Novagraaf International SA
Chemin de l'Echo 3
1213 Onex, Geneva (CH)**

(56) References cited:
WO-A1-2022/051546    CN-A- 114 091 603
CN-A- 114 496 083    CN-A- 114 496 099
CN-A- 115 223 662

• PHAM DUY ET AL: "stLearn: integrating spatial location, tissue morphology and gene expression to find cell types, cell-cell interactions and spatial trajectories within undissociated tissues", BIORXIV, 31 May 2020 (2020-05-31), XP093217017, Retrieved from the Internet <URL:https://www.biorxiv.org/content/10.1101/2020.05.31.125658v1> [retrieved on 20241022], DOI: 10.1101/2020.05.31.125658
• CHANG YUZHOU ET AL: "Define and visualize pathological architectures of human tissues from spatially resolved transcriptomics using deep learning", COMPUTATIONAL AND STRUCTURAL BIOTECHNOLOGY JOURNAL, vol. 20, 1 January 2022 (2022-01-01), Sweden, pages 4600 - 4617, XP093216715, ISSN: 2001-0370, DOI: 10.1016/j.csbj.2022.08.029
• BENJAMIN GRAHAM ET AL: "Submanifold Sparse Convolutional Networks", ARXIV.ORG, CORNELL UNIVERSITY LIBRARY, 201 OLIN LIBRARY CORNELL UNIVERSITY ITHACA, NY 14853, 5 June 2017 (2017-06-05), XP080767561

EP 4 376 010 B1

**Description**

FIELD OF THE TECHNOLOGY

Embodiments of this disclosure relate to the field of computer technologies, and in particular, to a data processing method and apparatus, a device, and a storage medium.

BACKGROUND OF THE DISCLOSURE

For some data, spatial location information of the data needs to be considered during data processing, for example, spatial transcriptomic data. The spatial transcriptomic data includes gene information and spatial location information of a cell. In a clustering scenario of the spatial transcriptomic data, to achieve clustering accuracy, clustering needs to be performed with reference to the gene information and the spatial location information of the cell.

However, in response to clustering the spatial transcriptomic data, a current data processing technology, for example, a clustering technology of the spatial transcriptomic data occupies a relatively large quantity of computing resources and has low efficiency.

Pham Duy ET AL: "stLearn: integrating spatial location, tissue morphology and gene expression to find cell types, cell-cell interactions and spatial trajectories within undissociated tissues", bioRxiv, 31 May 2020 (2020-05-31), XP093217017, DOI: 10.1101/2020.05.31.125658 provides a method for integrating spatial location, tissue morphology and gene expression to find cell types, cell-cell interactions and spatial trajectories within undissociated tissues.

Chang Yuzhou ET AL: "Define and visualize pathological architectures of human tissues from spatially resolved transcriptomics using deep learning", COMPUTATIONAL AND STRUCTURAL BIOTECHNOLOGY JOURNAL, vol. 20, 1 January 2022 (2022-01-01), pages 4600-4617, XP093216715, Sweden ISSN: 2001-0370, DOI: 10.1016/j.csbj.2022.08.029 provides a method to define and visualize pathological architectures of human tissues from spatially resolved transcriptomics using deep learning.

CN 114496099 A provides a cell function annotation method, including: obtaining sequencing data of a tissue sample, the sequencing data comprising gene expression data and spatial position data of each sequencing point, and the sequencing point corresponding to at least one cell; generating a gene sequencing matrix of the tissue sample based on the sequencing data, wherein the gene sequencing matrix is used for representing gene distribution conditions at different spatial positions in the tissue sample; based on the gene sequencing matrix, cell function annotation is carried out in a deep learning mode, and a cell function annotation result is obtained.

SUMMARY

The invention is set out in the claims annexed hereto.

BRIEF DESCRIPTION OF THE DRAWINGS

To describe the technical solutions in the embodiments of this disclosure more clearly, the following briefly introduces the accompanying drawings required for describing the embodiments. Apparently, the accompanying drawings in the following description show only some embodiments of this disclosure, and a person of ordinary skill in the art may still derive other accompanying drawings from these accompanying drawings without creative efforts.

FIG. 1 is a schematic diagram of an application scenario according to an embodiment of this disclosure.

FIG. 2 is a schematic diagram of clustering of spatial transcriptomic data.

FIG. 3 is a schematic diagram of a principle of scan equalization digital radiography (SEDR).

FIG. 4 is a schematic flowchart of a data processing method according to an embodiment of this disclosure.

FIG. 5 is a schematic diagram of a first image and a first feature map according to an embodiment of this disclosure.

FIG. 6A to FIG. 6D are schematic diagrams of an input image, a result of ordinary convolution, a result of ordinary sparse convolution, and a result of submanifold sparse convolution.

FIG. 7 is a schematic diagram of a data processing process according to an embodiment of this disclosure.

FIG. 8 is a schematic diagram of another data processing process according to an embodiment of this disclosure.

FIG. 9 is a schematic diagram of training a submanifold sparse convolution-based autoencoder according to an embodiment of this disclosure.

FIG. 10 is a schematic diagram of a processing process of spatial transcriptomic data according to an embodiment of this disclosure.

FIG. 11 is a schematic diagram of an annotation result on one sample in rat brain primary motor cortex data in multiplexed error-robust fluorescence in situ hybridization (MERFISH) according to this disclosure.

FIG. 12 is a schematic block diagram of a data processing apparatus according to an embodiment of this disclosure.

FIG. 13 is a schematic block diagram of an electronic device according to an embodiment of this disclosure.

DESCRIPTION OF EMBODIMENTS

The technical solutions in the embodiments of this disclosure are described below with reference to the accompanying drawings in the embodiments of this disclosure.

It is to be understood that in the embodiments of this disclosure, "B corresponding to A" indicates that B is associated with A. In an implementation, B may be determined according to A. However, it is to further be understood that, determining B according to A does not mean that B is determined according to A only, but B may also be determined according to A and/or other information.

In addition, in the descriptions of this disclosure, unless otherwise specified, "a plurality of" means two or more than two.

In addition, to clearly describe the technical solutions in the embodiments of this disclosure, terms such as "first" and "second" are used in embodiments of this disclosure to distinguish between same items or similar items that provide basically same functions or purposes. A person skilled in the art may understand that the terms such as "first" and "second" do not limit a quantity or an execution sequence, and the terms such as "first" and "second" do not indicate a definite difference.

For ease of understanding of the embodiments of this disclosure, related concepts in the embodiments of this disclosure are described below first.

Artificial intelligence (AI) is a theory, method, technology, and application system that uses a digital computer or a machine controlled by the digital computer to simulate, extend, and expand human intelligence, perceive an environment, determine knowledge, and use knowledge to determine an optimal result. In other words, AI is a comprehensive technology in computer science and attempts to understand the essence of intelligence and produce a new intelligent machine that can react in a manner similar to human intelligence. AI is to study the design principles and implementation methods of various intelligent machines, to enable the machines to have the functions of perception, reasoning, and decision-making.

The AI technology is a comprehensive discipline, and relates to a wide range of fields including both hardware-level technologies and software-level technologies. The basic AI technologies generally include technologies such as a sensor, a dedicated AI chip, cloud computing, distributed storage, a big data processing technology, an operating/interaction system, and electromechanical integration. AI software technologies mainly include several major directions such as a computer vision (CV) technology, a speech processing technology, a natural language processing technology, and machine learning/deep learning.

Machine learning (ML) is a multi-field interdisciplinary subject involving the probability theory, statistics, the approximation theory, convex analysis, the algorithm complexity theory, and the like, ML specializes in studying how a computer simulates or implements a human learning behavior to determine new knowledge or skills, and reorganize an existing knowledge structure to keep improving its performance. The ML, as the core of AI, is a basic way to make the computer intelligent, and is applicable to various fields of AI. ML and deep learning generally include technologies such as an artificial neural network, a belief network, reinforcement learning, transfer learning, inductive learning, and learning from demonstrations.

With the research and progress of the AI technology, the AI technology is studied and applied in a plurality of fields such as a common smart home, a smart wearable device, a virtual assistant, a smart speaker, smart marketing, unmanned driving, automatic driving, an unmanned aerial vehicle, a robot, smart medical care, and smart customer service. It is believed that with the development of technologies, the AI technology will be applied to more fields, and play an increasingly important role.

In the embodiments of this disclosure, data processing is implemented by means of the AI technology, for example, processing such as clustering, type annotation, and downstream analysis on to-be-processed data is implemented.

First, an application scenario of this embodiment of this disclosure is described.

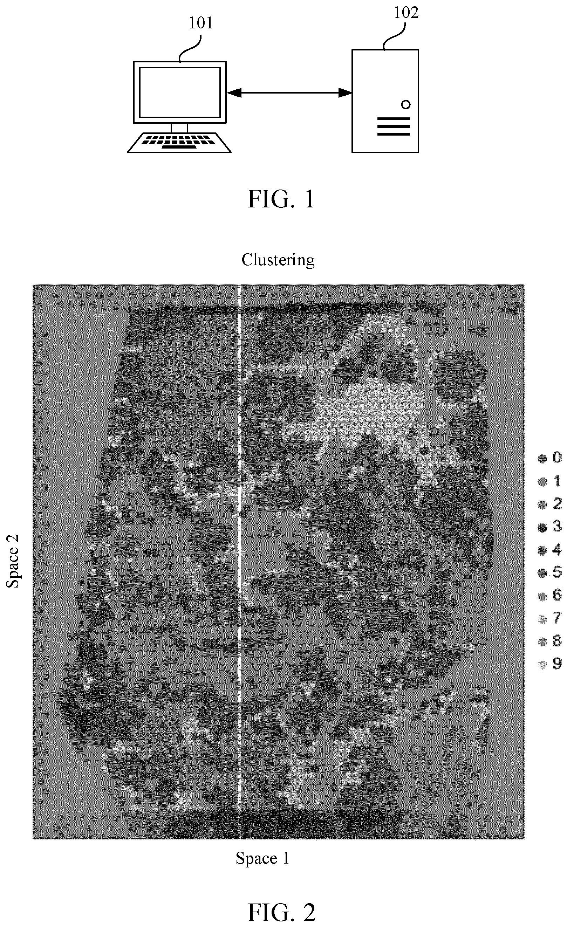

FIG. 1 is a schematic diagram of an application scenario according to an embodiment of this disclosure, including a terminal device 101 and a server 102.

The terminal device 101 may include, but not limited to, a personal computer (PC), a PDA (a tablet computer), a mobile phone, a wearable intelligent device, a medical device, and the like. The device is generally provided with a display apparatus. The display apparatus may alternatively be a display, a display screen, a touch screen, or the like. The touch screen may alternatively be a touch screen, a touch panel, or the like. The display apparatus may be configured to display a processing result and the like.

There may be one or more servers 102. In response to a plurality of servers 102, at least two servers are configured to provide different services, and/or at least two servers are configured to provide a same service, for example, provide the same service in a load balancing manner. This is not limited in this embodiment of this disclosure. The server 102 may be an independent physical server, or may be a server cluster or a distributed system formed by a plurality of physical servers, or may be a cloud server that provides a basic cloud computing service such as a cloud service, a cloud database, cloud computing, a cloud function, cloud storage, a network service, cloud communication, a middleware service, a domain name service, a security service, a content delivery network (CDN), big data, and an artificial intelligence platform. The server 102 may alternatively be a node of a blockchain.

The terminal device 101 and the server 102 may be directly or indirectly connected in a wired or wireless communication manner. This is not limited in this embodiment of this disclosure.

In some embodiments, the server 102 in this embodiment of this disclosure may train a feature extraction model (for example, a submanifold sparse convolution-based autoencoder) in the embodiments of this disclosure and store the trained feature extraction model.

In some embodiments, the data processing method in the embodiments of this disclosure may be completed by the terminal device 101. For example, the terminal device 101 determines to-be-processed data and determines a processing result of the to-be-processed data according to the method provided in the embodiments of this disclosure. In an example, the terminal device 101 loads the trained feature extraction model in the server 102, performs feature extraction on a first image by using the feature extraction model, to determine second feature information of each object in N objects, and performs preset processing on the second feature information of each cell in the N cells, to determine the processing result of the to-be-processed data.

In some embodiments, the data processing method in the embodiments of this disclosure may be completed by the server 102. For example, the terminal device 101 sends to-be-processed data to the server 102, and the server 102 processes the to-be-processed data according to the method provided in the embodiments of this disclosure, to determine a processing result of the to-be-processed data. In an example, the server 102 loads the trained feature extraction model stored in the server 102, performs feature extraction on a first image corresponding to the to-be-processed data, to determine second feature information of each object in N objects, and performs preset processing on the second feature information of each cell in the N cells, to determine the processing result of the to-be-processed data. In some embodiments, the server 102 may further send the processing result to the terminal device 101 for display.

In some embodiments, the data processing method in the embodiments of this disclosure may be jointly completed by the terminal device 101 and the server 102. For example, the server 102 performs an operation related to a network model, and the terminal device 101 performs another operation than the operation related to the network model. For example, the terminal device 101 determines to-be-processed data, converts the to-be-processed data into a first image, and sends the first image to the server 102. The server 102 performs feature extraction on the first image, for example, performs feature extraction on the first image by using the trained feature extraction model, to determine second feature information of each object in N objects. Then, the server performs preset processing on the second feature information of each cell in the N cells, to determine a processing result of the to-be-processed data, and finally sends the processing result to the terminal device 101 for display.

The application scenario in this embodiment of this disclosure includes, but not limited to, that shown in FIG. 1.

The data processing method provided in this embodiment of this disclosure is applicable to any data processing application that needs to combine spatial location information.

In some embodiments, this embodiment of this disclosure is applicable to a clustering scenario of spatial transcriptomic data.

A spatial transcriptome sequencing technology is a new technology in recent years, and the key of the spatial transcriptome sequencing technology lies in that in addition to gene information of a cell, spatial location information of the cell that is not available for single cell transcriptomic data can also be determined.

A main objective of clustering analysis of the spatial transcriptomic data is to divide a group of cells into a plurality of groups according to gene information of each cell. Currently, a general clustering analysis process is mainly divided into two steps: First, feature extraction is performed, and then clustering is performed on an extracted feature by using a clustering algorithm. The clustering algorithm may be a general clustering algorithm such as a K-means clustering algorithm (K-means) or a K-nearest neighbor (KNN) algorithm, or a clustering algorithm such as Louvain or Leiden based on community detection that is more suitable for cell clustering. For example, FIG. 2 is a schematic diagram of clustering of spatial transcriptomic data.

A core of clustering of the spatial transcriptomic data is a feature extraction process. A group of cell data generally includes tens of thousands of different genes. Expression of each gene is directly used as an input of a clustering algorithm, which occupies an extremely large quantity of computing resources and includes a lot of useless information (not all genes are meaningful for cell clustering, for example, gene information "Housekeeping" existing in all the cells is not meaningful for clustering). In addition, for the spatial transcriptomic data, spatial location information is a key to differentiate from single-cell data because single-cell transcriptomic data has only gene information of a cell and does not know an absolute or relative location of the cell, while the spatial transcriptomic data is data that includes both the gene information of the cell and knows the location of the cell. Therefore, for clustering analysis of the spatial transcriptomic data, how to effectively encode spatial location information into a feature is important content of a feature extraction process.

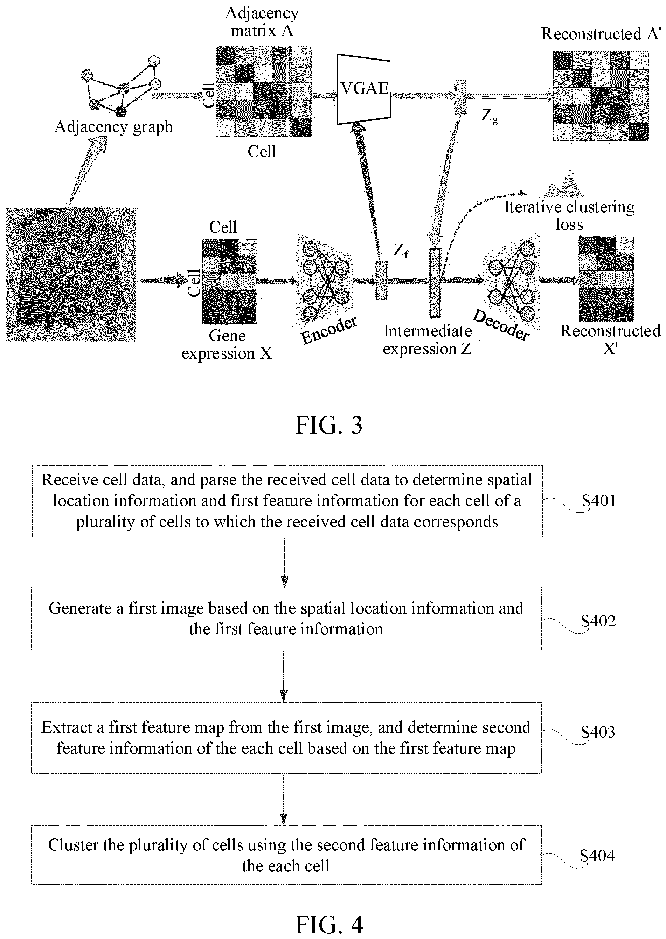

Currently, the spatial location information is encoded into feature information by using a scan equalization digital radiography (SEDR) method. Specifically, as shown in FIG. 3, spatial transcriptomic data is first parsed to determine a domain matrix and a gene information matrix, then the domain matrix is encoded by using a variational graph auto-encoder (VGAE), to determine a feature $Z_g$ that may represent spatial information, and the gene information matrix is encoded by using an encoder, to determine a feature $Z_f$ that may represent gene information. The feature $Z_g$ that may represent the spatial information is merged into the feature $Z_f$ that may represent the gene information, to determine intermediate expression Z. The intermediate expression Z includes both the spatial information and the gene information, and finally a subsequent task such as clustering can be implemented by using the intermediate expression Z.

It can be learned from FIG. 3 that a neighborhood relationship between cells is mainly used in response to the spatial information being encoded by using the SEDR, and if too few neighborhoods are considered in response to each cell being encoded, spatial location information cannot be effectively used. Therefore, to ensure effectiveness, a relatively large quantity of neighborhoods are considered, but considering the relatively large quantity of neighborhoods exponentially increases occupied computing resources, and the entire data processing process is relatively complex and has low efficiency.

To resolve the foregoing technical problems, in the embodiments of this disclosure, cell data is received, and the received cell data is parsed to determine spatial location information and first feature information for each cell of a plurality of cells to which the received cell data corresponds; a first image is generated based on the spatial location information and the first feature information; a first feature map is extracted from the first image, and second feature information of the each cell is determined based on the first feature map. In this way, preset processing (for example, cell clustering or cell annotation) is performed by using the second feature information of the N cells, to determine an accurate processing result. That is, in the embodiments of this disclosure, the to-be-processed data is used to generate the first image, the first image including the spatial location information and the first feature information of each cell in the N cells, then feature extraction is performed on the first image, to determine the second feature information of each cell in the N cells, to encode the spatial location information into a feature, and preset processing such as clustering is directly performed on the second feature information including the spatial location information. The entire data processing process is simple, occupies fewer computing resources, and has high data processing efficiency. In addition, in the embodiments of this disclosure, in response to processing the to-be-processed data, the to-be-processed data is used to generate the first image, and feature extraction is performed on the first image. In response to performing feature extraction on the first image, only the second feature information of each cell in the N cells is extracted. If the first image includes a pixel whose pixel value is zero, feature extraction is not performed on the pixel whose pixel value is zero in the first image, to further save the computing resources and improve the data processing efficiency.

The technical solution of the embodiments of this disclosure is described in detail below through some embodiments. The following embodiments may be mutually combined, and same or similar concepts or processes may not be repeatedly described in some embodiments.

FIG. 4 is a schematic flowchart of a data processing method according to an embodiment of this disclosure.

An execution body of this embodiment of this disclosure may be an apparatus having a data processing function, for example, a data processing apparatus. In some embodiments, the data processing apparatus may be a server. In some embodiments, the data processing apparatus may be a terminal device. In some embodiments, the data processing apparatus may be a system formed by the server and the terminal device. Both the server and the terminal device may be understood as an electronic device. Therefore, for ease of description, an example in which the execution body is the electronic device is used for description.

As shown in FIG. 4, the data processing method in this embodiment of this disclosure includes the following steps.

S401. Receive cell data, and parse the received cell data to determine spatial location information and first feature information for each cell of a plurality of cells to which the received cell data corresponds.

In embodiments of this disclosure, there are N objects to be processed. N is a positive integer.

In some embodiments, N is a positive integer greater than 1, that is, there are a plurality of research objects in this embodiment of this disclosure.

A specific type of the object is a cell having spatial location information. In some embodiments of this disclosure, a cell may be specified as a sub-cell having spatial location information, or a cell cluster having spatial location information.

The to-be-processed data may be understood as cell data corresponding to the N cells. The cell data includes or implicitly includes the spatial location information and the first feature information of each cell in the N cells.

In some embodiments, the cell data may be generated by the electronic device.

In some embodiments, the cell data may be sent by another device.

In some embodiments, the cell data may alternatively be read by the electronic device from a storage device. For example, in response to receiving a processing request of the cell data, the electronic device reads the cell data from the storage device in response to the request.

A specific method for receiving the cell data is not limited in this embodiment of this disclosure.

To accurately analyze and process the cell data, in this embodiment of this disclosure, after the cell data is received, the cell data is parsed, to determine the spatial location information and the first feature information of each cell in the N cells.

That is, in this embodiment of this disclosure, each cell in the N cells includes the spatial location information and the first feature information.

For example, each cell in the N cells has different spatial location information.

The spatial location information may be understood as any information that may represent a spatial location of an object, for example, the spatial location information is location coordinates of a center point of the object.

For example, the first feature information of each cell in the N cells may be the same or may be different, or may be partially the same or may be partially different. This is not limited in this embodiment of this disclosure.

A specific type of the first feature information is not limited in this embodiment of this disclosure.

In some embodiments, first feature information of a cell may be understood as original feature information of the cell included in the cell data, and the original feature information may be understood as inherent feature (attribute) data of the cell. For example, the first feature information of a cell may refer to gene information of the cell. The gene information include a gene type that each cell belongs to.

The following describes a process of determining the spatial location information and the first feature information.

A specific implementation of parsing the cell data to determine the spatial location information and the first feature information of each cell in the N cells in S401 is not limited.

In some embodiments, if the cell data includes the spatial location information and the first feature information of each cell in the N cells, the spatial location information and the first feature information of each cell in the N cells may be directly read from the cell data.

In some embodiments, at least one sub-object in this embodiment of this disclosure may correspond to one object. For example, the object is a cell, and at least one sequencing point may be configured on the cell, and the at least one sequencing point is used to capture gene information of the cell. In this way, one sequencing point may be recorded as one sub-object corresponding to the cell.

In this embodiment, it is assumed that the cell data includes first sub-feature information captured by a plurality of sub-objects, each sub-object in the plurality of sub-objects may correspond to one cell in the N cells, and the first sub-feature information may be all or a part of the first feature information. Based on the foregoing, the plurality of sub-objects in the cell data may correspond to the N cells. For example, a sub-object 11 and a sub-object 13 in the cell data correspond to a cell 1 in the N cells, and a sub-object 21 and a sub-object 22 in the cell data correspond to a cell 2 in the N cells. In this way, first sub-feature information captured by the sub-object 11 and the sub-object 13 may be used as first feature information of the cell 1, and first sub-feature information captured by the sub-object 21 and the sub-object 22 may be used as first feature information of the cell 2. Referring to the method, the first feature information of each cell in the N cells may be determined.

In an implementation of this embodiment, the cell data includes a correspondence between the plurality of sub-objects and the N cells. In this way, the electronic device may enable the plurality of sub-objects included in the cell data to correspond to the N cells according to the correspondence between the plurality of sub-objects in the cell data and the N cells, and use first sub-feature information corresponding to each cell in the N cells as first feature information of the object.

In this embodiment, a method for determining the spatial location information of each cell in the N cells includes at least the following examples.

Example 1: the cell data includes the spatial location information of each cell in the N cells. In this way, the electronic device may directly determine the spatial location information of each cell in the N cells from the cell data.

Example 2: the cell data includes spatial location information of a sub-object. In this way, the electronic device may determine the spatial location information of the N cells according to spatial location information of a sub-object corresponding to each cell in the N cells. For example, for an $i^{th}$ cell in the N cells, a sub-object corresponding to the $i^{th}$ cell is a sub-object i1 and a sub-object i2, and spatial location information of the $i^{th}$ cell may be determined according to spatial location information of the sub-object i1 and the sub-object i2. For example, an average value of the spatial location information of the sub-object i1 and the sub-object i2 is determined as the spatial location information of the $i^{th}$ cell.

A specific representation form of the spatial location information is not limited in this embodiment of this disclosure.

In some embodiments, the spatial location information is represented in a form of a matrix.

For example, the spatial location information of the N cells is the following matrix A:

$$A=\{(x_1, y_1), (x_2, y_2), ... (x_i, y_i), ... (x_N, y_N)\}$$

$(x_i, y_i)$ represents the spatial location information of the $i^{th}$ cell in the N cells.

A specific representation form of the first feature information is not limited in this embodiment of this disclosure.

In some embodiments, the first feature information is represented in a form of a matrix.

For example, the first feature information of the N cells is the following matrix B:

$$B=\{(a_{11}, a_{12}, ..., a_{1G}), (a_{21}, a_{22}, ..., a_{2G}), ..., (a_{i1}, a_{i2}, ..., a_{iG}), ..., (a_{N1}, a_{N2}, ..., a_{NG})\}.$$

$(a_{i1}, a_{i2}, ..., a_{iG})$ represents G pieces of first feature information of the $i^{th}$ cell in the N cells. At least one piece of first feature information in the G pieces of first feature information of the $i^{th}$ cell may be empty. That is, G may be understood as total types that the first feature information of the N cells belongs to, and types of the first feature information of various cells in the N cells may be the same or may be different. That is, some cells in the N cells may have different types of first feature information in the G pieces of first feature information.

In this embodiment of this disclosure, after determining the cell data according to the method, and parsing the cell data to determine the spatial location information and the first feature information of each cell in the N cells, the electronic device performs the following S402.

S402. Generate a first image based on the spatial location information and the first feature information.

In this embodiment of this disclosure, generating the first image includes fusing the first feature information and the spatial location information of all N cells into the first image. For example, one way of generating is to convert the spatial location information and the first feature information of each cell in the N cells into location information and color channel information of each pixel in the first image. The correspondence between the cell and the pixel in the first image may be recorded, and then may be used in S403. Color channel information is used to store the color information of an image, one color channel corresponding to one color mode. For example, for a RGB image, each pixel has 3 color channels for red, green and blue, respectively.

In this embodiment of this disclosure, the cell data is converted into the first image, the first image including the spatial location information and the first feature information of each cell in the N cells. Therefore, feature extraction is performed on the first image, to determine second feature information of the N cells, the second feature information inherently fusing the spatial location information and the first feature information. In response to performing subsequent data processing based on the second feature information, a data processing effect can be improved.

In addition, in this embodiment of this disclosure, the first feature information and the spatial location information are fused into the first image, and feature extraction is directly performed on the first image in which the spatial location information and the first feature information are fused, to encode the spatial location information into a feature. Compared with a manner of extracting a domain matrix, encoding the domain matrix, to determine spatial encoding information, and fusing the spatial encoding information into a feature in SEDR, in this embodiment of this disclosure, the entire data processing is simple, and occupies fewer computing resources. Further, in this embodiment of this disclosure, the spatial location information of the N cells is directly used as spatial information, which can represent spatial information of a cell more accurately than a domain relationship of the cell. Further, data processing accuracy is improved, a data volume of the spatial information in this disclosure is relatively small, and the occupied computing resources can be further reduced.

That is, in this embodiment of this disclosure, the generated first image fuses the spatial location information and the first feature information of each cell in the N cells. For example, each cell in the N cells is used as a pixel whose pixel value is not zero in the first image, and the first feature information of the cell is used as color channel information of the first image, so as to form the first image

Specifically, each cell corresponds to a pixel in the first image (in this case, the first image is a blank image or an initial image) based on the spatial location information of each cell in the N cells, and the first feature information of each cell is used as color channel information of the pixel corresponding to each cell, so that the cell data is converted into the first image, the converted first image including the spatial location information and the first feature information of each cell in the cell data.

A specific process of converting the cell data into the first image in S402 is not limited in this embodiment of this disclosure.

**In** some embodiments, each cell in the N cells is used as a pixel in the first image according to the spatial location information of each cell in the N cells, and the first feature information is used as color channel information of the first image, to determine the first image including N pixels.

**In** an example, the first image does not include a pixel whose pixel value is zero.

For example, N is 1000, and the first image includes 1000 pixels. Each pixel represents each cell in 1000 cells, and sorting of the 1000 cells in the first image is determined according to spatial location information of the 1000 cells. It is assumed that spatial location information of each cell is two-dimensional coordinates, for example, (x, y). The 1000 cells are arranged on a two-dimensional plane according to the spatial location information of each cell, and there is no gap among the 1000 cells. Each cell in the sorted 1000 cells is used as a pixel in the first image, to determine the first image including 1000 pixels. In this embodiment, a method for sorting the 1000 cells according to the spatial location information is not limited. For example, the 1000 cells are first sorted according to sizes of x coordinates of the 1000 cells. If the x coordinates are the same, cells whose x coordinates are the same are sorted according to sizes of y coordinates, so that the 1000 cells are arranged on the two-dimensional plane.

**In** this embodiment, the determined first image may be a rectangle or may not be a rectangle. This is not limited in this embodiment of this disclosure.

**In** this embodiment, the first image does not include an invalid pixel whose pixel value is zero. Therefore, In response to performing feature extraction on the first image, all valid information is extracted, so that reliability of feature extraction is improved, and computation is not performed on the invalid pixel whose pixel value is zero, thereby saving the computing resources.

In some embodiments, the cell data may alternatively be converted into the first image through the following steps S402-A and S402-B.

S402-A. Create a blank second image.

S402-B. Determine, according to the spatial location information of the each cell, a pixel in the blank second image corresponding to the each cell, to obtain the first image, wherein the first feature information is used as color channel information of the pixel.

In this embodiment, a blank second image is first created, that is, all pixels in the second image are pixels whose pixel values are zero. Then, the N cells are filled into corresponding locations in the blank second image one by one according to the spatial location information of each cell in the N cells. For example, for an $i^{th}$ cell in the N cells, the $i^{th}$ cell is filled into a location corresponding to spatial location information of the $i^{th}$ cell in the second image, and so on, and the N cells may be filled into the blank second image. The filling means the value of the respective pixel becomes non-zero.

Then the first image may be determined by using the first feature information as color channel information of the first image.

A specific size of the second image is not limited in this embodiment of this disclosure provided that it is ensured that the second image includes at least N pixels.

In some embodiments, if a quantity of pixels included in the second image is greater than N, the first image generated according to the second image includes N pixels whose pixel values are not zero and at least one pixel whose pixel value is zero, the N pixels whose pixel values are not zero being in one-to-one correspondence with the N cells.

A specific shape of the second image is not limited in this embodiment of this disclosure.

In some embodiments, the second image may be a rectangle.

In some embodiments, the second image may be a regular shape except the rectangle, for example, a circle, an ellipse, or a polygon, or may be an irregular shape.

The following describes a process of creating the blank second image in S402-A.

A specific manner of creating the blank second image is not limited in this embodiment of this disclosure.

In some embodiments, a size and a shape of the second image are preset.

In some embodiments, the second image is a rectangle or square image including at least N pixels.

In some embodiments, S402-A includes the following steps.

S402-A1. Create the blank second image according to the spatial location information of each cell in the N cells.

In this embodiment, the blank second image is created according to the spatial location information of each cell in the N cells, so that each cell in the N cells may correspond to a pixel in the second image.

In this embodiment, the N cells may be relatively sparse, that is, a distance between cells in the N cells is relatively large, for example, at least a few units. Therefore, if the spatial location information is not processed, a minimum distance between pixels whose pixel values are not zero in the formed first image is a pixels.

In a possible implementation of S402-A1, to reduce a size of the first image, in response to the blank second image being created, the spatial location information of the N cells may be reduced by several times, and the second image is created according to the spatial location information that is reduced by several times. For example, a maximum difference between x and a maximum difference between y of the spatial location information that is reduced by several times of the N cells are determined, and the second image whose length is H and width is W is created by using the maximum difference between x as the length H of the second image and the maximum difference between y as the width W of the second image, a quantity of color channels of the second image being total types G that the first feature information of the N cells belongs to. In this implementation, a size of the created blank second image is less than a size of a minimum rectangle of the N cells. Therefore, in response to filling the N cells into the second image, to form the first image, a quantity of pixels whose pixel values are zero included in the formed first image is relatively small.

That there are a units between the N cells is only an example, and the distance depends on an actual condition.

In a possible implementation of S402-A1, S402-A1 includes the following steps.

S402-A11. Determine a minimum rectangle enclosing the N cells according to the spatial location information of each cell in the N cells.

S402-A12. Create the blank second image by using a size of the minimum rectangle as a size of the second image.

In this implementation, the blank second image is directly created according to the spatial location information of each cell in the N cells without scaling the spatial location information, thereby simplifying the process of creating the second image and ensuring that the N cells can be filled into the blank second image without overlapping.

Specifically, a minimum value and a maximum value of coordinates of the N cells in an x-axis direction are determined, and a difference between the minimum value and the maximum value in the x-axis direction is determined as a length H of the minimum rectangle. Similarly, a minimum value and a maximum value of coordinates of the N cells in a y-axis direction are determined, and a difference between the minimum value and the maximum value in the y-axis direction is determined as a width W of the minimum rectangle, so that the minimum rectangle of the N cells is determined as a rectangle whose length is H and width is W. Then, the blank second image whose length is H and width is W is created by using a size of the minimum rectangle of the N cells as a size of the second image.

In this implementation, the size of the created second image is consistent with the size of the minimum rectangle of the N cells. In this way, it can be ensured that each cell in the N cells corresponds to a pixel in the second image. Then, the N cells are filled into the blank second image one by one according to the spatial location information of each cell in the N cells. For example, for an $i^{th}$ cell in the N cells, it may be determined that the $i^{th}$ cell corresponds to a $j^{th}$ pixel in the second image according to spatial location information of the $i^{th}$ cell, so that the $i^{th}$ cell may be filled into the $j^{th}$ pixel in the second image, and so on, and the N cells may be filled into the blank second image according to the spatial location information of each cell in the N cells to generate the first image.

For example, it is assumed that the spatial location information of the N cells is an N*2-dimensional matrix A, which is recorded as center coordinates of each cell in the N cells. It is assumed that the first feature information of the N cells is an N*G-dimensional matrix B, which is recorded as G types that first feature information of the N cells belongs to. For example, an $i^{th}$ row and a $j^{th}$ column of the matrix B represent an amount of $j^{th}$ first feature information of an $i^{th}$ cell. Referring to the foregoing embodiments, the blank second image is first created according to the spatial location information of each cell in the N cells, that is, the spatial location matrix B. The second image may be understood as a matrix of H*W*G, G representing a quantity of color channels. A color channel may represent a type of gene, in response to the first feature information being gene information. Then, the N cells are filled into the second image according to the spatial location information of each cell in the N cells to determine the first image. For example, a value of a $g^{th}$ color channel in an $h^{th}$ row and a $w^{th}$ column, corresponding to a pixel of (h, w, g) in the first image, represents an amount of a $g^{th}$ first feature information of a cell whose center coordinates are (h, w).

According to the method, in the generated first image, each cell in the N cells occupies a pixel, and the pixel occupied by the cell is recorded as a pixel whose pixel value is not zero. All color channels of the pixel whose pixel value is not zero form all first feature information of the cell. A pixel whose pixel value is zero in the first image represents that there is no cell at the location, and values of all color channels of the pixel whose pixel value is zero are 0. Therefore, in the generated first image of H*W, only the N pixels whose pixel values are not zero include information, and another pixel whose pixel value is zero does not include information.

For example, it is assumed that the generated first image is represented by using a matrix C as follows:

$$C = \begin{bmatrix} \odot & \otimes & \dots & \odot \\ \otimes & \odot & \dots & \otimes \\ \dots & \dots & \dots & \dots \\ \odot & \odot & \dots & \otimes \end{bmatrix}_{H \times W}$$

⊙ represents a pixel whose pixel value is zero, and ⊗ represents a pixel whose pixel value is not zero, that is, a pixel in which a cell is located.

**In** this embodiment of this disclosure, after the cell data is converted into the first image according to the step, the following step S403 is performed.

S403. Extract a first feature map from the first image, and determine second feature information of the each cell based on the first feature map.

It can be learned from the foregoing that in this embodiment of this disclosure, the cell data is converted into the first image based on the spatial location information and the first feature information of the N cells, the first image including the spatial location information and the first feature information of each cell in the N cells. Then, feature extraction is performed on the first image, to determine the second feature information of each cell in the N cells, so that the generated second feature information fuses the spatial location information and the first feature information of the N cells, and in response to performing subsequent data processing by using the second feature information, data processing accuracy can be improved.

That is, in this embodiment of this disclosure, the second feature information may be understood as a feature vector determined by performing feature extraction on the first image, the feature vector fusing spatial location information and first feature information of a cell.

A specific implementation of extracting the second feature information of each cell in the N cells from the first image in S403 is not limited in this embodiment of this disclosure.

In some embodiments, it can be learned from the foregoing that if the first image includes N pixels whose pixel values are not zero and does not include a pixel whose pixel value is zero, feature extraction may be performed on the first image by using any feature extraction method, to determine the second feature information of each cell in the N cells. Because the first image does not include the pixel whose pixel value is zero, there is no waste of computing resources caused by processing an invalid pixel whose pixel value is zero, thereby saving the computing resources.

In some embodiments, it can be learned from the foregoing that in addition to including the N pixels whose pixel values are not zero, the first image further includes at least one pixel whose pixel value is zero, for example, as shown in the matrix C. In this embodiment, to save the computing resources, the electronic device performs feature extraction on only the pixel whose pixel value is not zero in the first image, and does not perform feature extraction on the pixel whose pixel value is zero in the first image, to determine the second feature information of each cell in the N cells.

A specific implementation of performing feature extraction on the pixel whose pixel value is not zero in the first image and skipping performing feature extraction on the pixel whose pixel value is zero in the first image is not limited in this embodiment of this disclosure.

In a possible implementation of this embodiment, S403 includes the following steps S403-A and S403-B.

S403-A. Perform feature extraction on the first image, to determine a first feature map.

The first feature map includes N non-zero elements in one-to-one correspondence with the N cells.

In this implementation, feature extraction is performed on the first image, to determine a first feature map, the first feature map including N non-zero elements, and the N non-zero elements being in one-to-one correspondence with the N cells, that is, the N non-zero elements of the first feature map being in one-to-one correspondence with the N pixels whose pixel values are not zero in the first image. And as described in the above S402, the correspondence between the cell and the pixel in the first image may be recorded. Hence, the N non-zero elements of the first feature map can be mapped to the N cells.

In this step, a size of the determined first feature map may be the same as or may be different from a size of the first image. This is not limited in this embodiment of this disclosure.

In some embodiments, the size of the first feature map determined in this step is the same as the size of the first image, a location of a zero element in the first feature map is consistent with a location of a pixel whose pixel value is zero in the first image, and a location of a non-zero element in the first feature map is consistent with a location of a pixel whose pixel value is not zero in the first image.

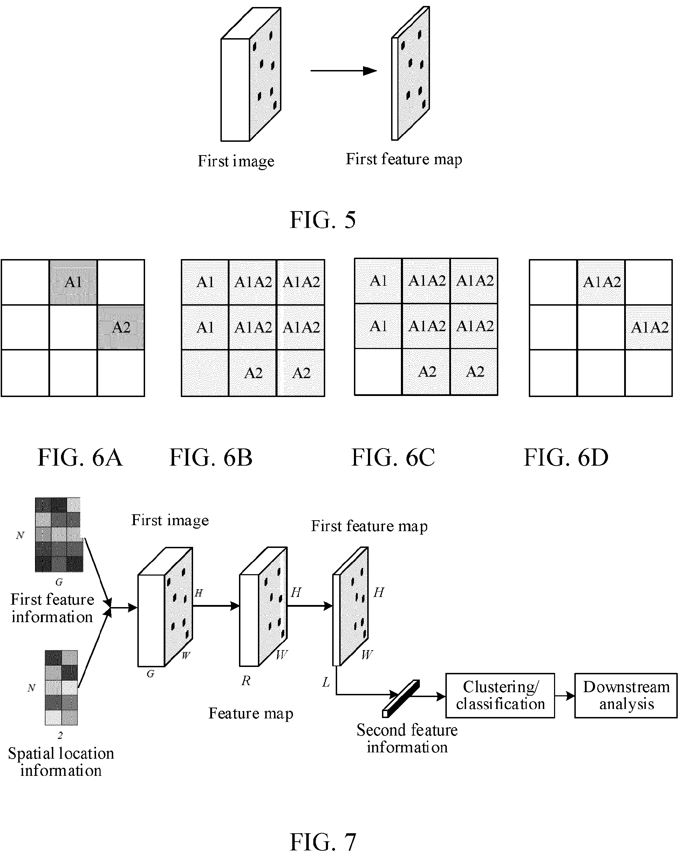

For example, as shown in FIG. 5, a left side in FIG. 5 is a first image, a black square in the first image represents a cell or a pixel whose pixel value is not zero, and there are a total of seven cells. A right side in FIG. 5 is a first feature map, a size of the first feature map is consistent with a size of the first image, a black square in the first feature map represents a non-zero element, and there are a total of seven non-zero elements. locations of the seven non-zero elements are consistent with locations of the seven pixels (corresponding to seven cells) in the first image, and a quantity and locations of zero elements in the first feature map are also consistent with a quantity and locations of pixels whose pixel values are zero in the first image. It can be learned from FIG 5 that in this embodiment of this disclosure, in response to performing feature extraction on the first image, to generate the first feature map, feature extraction is performed on only pixels whose pixel values are not zero (that is, cells) in the first image, and feature extraction is not performed on a pixel whose pixel value is zero in the first image, thereby avoiding processing invalid information, further saving the computing resources, and improving data processing efficiency.

A specific manner of extracting the first feature map from the first image in S403-A is not limited in this embodiment of this disclosure.

In some embodiments, feature extraction is performed on the first image by using a submanifold sparse convolution-based autoencoder, to determine the first feature map.

Submanifold sparse convolution (SubMConv) is also referred to as a valid sparse convolution, and feature extraction on the pixel whose pixel value is zero in the first image may be skipped.

The following compares the submanifold sparse convolution with ordinary convolution and ordinary sparse convolution.

FIG. 6A to FIG. 6D show a difference among ordinary convolution, ordinary sparse convolution, and submanifold sparse convolution. FIG. 6A is an input sparse image. As shown in FIG. 6A, the sparse image includes two pixels A1 and A2 whose pixel values are not zero, and others are all pixels whose pixel values are zero. FIG. 6B to FIG. 6D are respectively outputs after the input image shown in FIG. 6A is processed through ordinary convolution, ordinary sparse convolution, and submanifold sparse convolution in response to a size of a convolution kernel being 3. In FIG. 6B to FIG. 6D, blank light gray cells indicate no information, cells with text indicate that information is included, and white cells indicate that no storage is required at the locations.

In response to performing a convolution operation by using a convolution kernel with a size of 3, which is equivalent to performing convolution on each cell, information of the cell and a total of at most nine cells around the cell is processed. FIG. 6A is used as an example. In response to performing convolution on a cell at the bottom left corner, there is no effective information that needs to be processed. Therefore, a value of 0 is returned. In ordinary convolution, the value of zero needs to be stored. Therefore, information at the location needs to be used as an input in response to performing a next round of convolution. In ordinary sparse convolution, the value of zero does not participate in the operation. Therefore, the location does not need to be stored in response to returning the value of 0 is ed. In submanifold sparse convolution, the value of zero does not participate in the operation, and only content at a location corresponding to a non-zero value in an original input is saved. Therefore, after the submanifold sparse convolution is performed, sizes of an output and an input are completely consistent with a non-zero value location and a zero value location. It can be learned that the use of the submanifold sparse convolution greatly reduces content that participates in computation and needs to be stored, thereby greatly reducing computing performance requirements and occupation of computing resources.

A quantity of submanifold sparse convolution layers included in a submanifold sparse convolution-based autoencoder is not limited in this embodiment of this disclosure. For example, there is at least one submanifold sparse convolution layer.

In some embodiments, the submanifold sparse convolution-based autoencoder includes two submanifold sparse convolution layers. In this case, the data processing process provided in this embodiment of this disclosure may be represented as that shown in FIG. 7.

Specifically, as shown in FIG. 7, cell data is determined, and the cell data is parsed to determine spatial location information and first feature information of each cell in N cells, the spatial location information of the N cells being an N*2-dimensional matrix, and the first feature information of the N cells being an N*G-dimensional matrix. Then, the cell data is converted into a first image based on the spatial location information and the first feature information of each cell. For example, as shown in FIG. 7, a size of the first image is H*W, and a quantity of color channels is G. The first image is inputted into the submanifold sparse convolution-based autoencoder, and is compressed through a first submanifold sparse convolution layer in a color channel direction, to determine a feature map of H*W*R. After the feature map of H*W*R is compressed through a second submanifold sparse convolution layer, a first feature map of H*W*L is generated. That is, in this embodiment of this disclosure, for encoding layers in the submanifold sparse convolution-based autoencoder, the first image is compressed by using two submanifold sparse convolution layers in a direction of a quantity of color channels, and after two submanifold sparse convolutions are performed, an input X with a size of H*W*G is encoded into a first feature map with a size of H*W*L.

In some embodiments, in this embodiment of this disclosure, the submanifold sparse convolution-based autoencoder may include a decoding layer, the decoding layer including at least one submanifold sparse deconvolution layer.

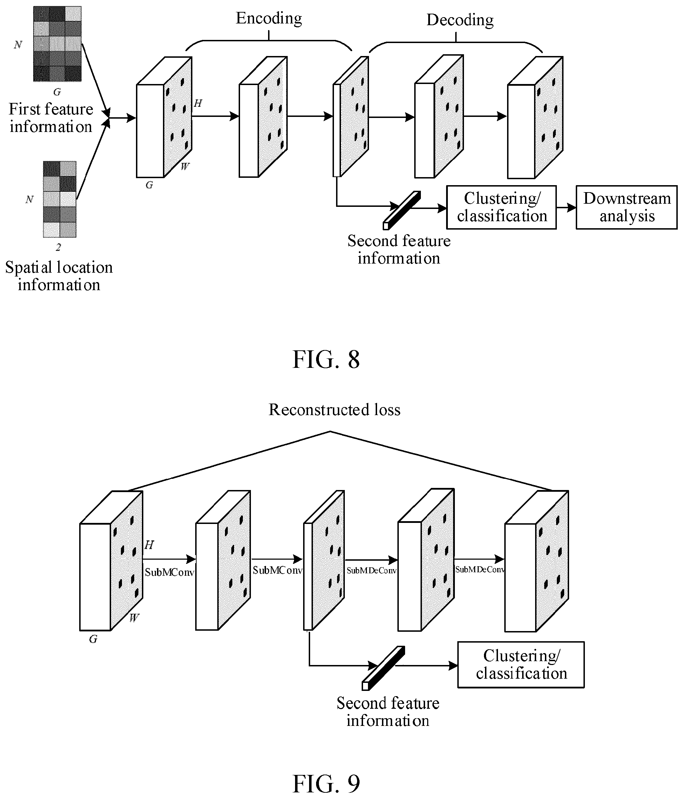

In an example, as shown in FIG. 8 and FIG. 9, the submanifold sparse convolution-based autoencoder includes an encoding layer and a decoding layer, the encoding layer including two submanifold sparse convolution (SubMConv) layers, and the decoding layer including two submanifold sparse deconvolution (SubDeMConv) layers. In response to the submanifold sparse convolution-based autoencoder being trained, the encoding layer compresses a training image by using the two submanifold sparse convolution layers in a color channel direction, to encode an input X with a size of H*W*G into a feature map with a size of H*W*L. Then, the decoding layer decodes the feature map with the size of H*W*L again by using the two submanifold sparse deconvolution layers to a reconstructed matrix X' with a size of H*W*G. Then, a reconstructed loss between X and X' is optimized, to train the submanifold sparse convolution-based autoencoder.

In some embodiments, the submanifold sparse convolution-based autoencoder may use a relatively large convolution kernel, to ensure that information about a specific quantity of surrounding cells can be considered during each sparse convolution.

In some embodiments, the submanifold sparse convolution-based autoencoder may include only the encoding layer and does not include the decoding layer. In this case, the encoding layer may be trained by introducing a label or another guidance.

During actual use, feature extraction is performed on the first image by using the encoding layer in the submanifold sparse convolution-based autoencoder, to determine a first feature map.

According to the method, after feature extraction is performed on the first image, to determine the first feature map, the following step S403-B is performed.

S403-B. Determine the second feature information of each cell in the N cells according to feature information of the N non-zero elements in the first feature map.

It can be learned from the foregoing that the generated first feature map includes feature information of the N non-zero elements in one-to-one correspondence with the N non-zero pixels in the first image, and the correspondence between the cell and the pixel in the first image may be recorded when generating the first image in S402. Hence, the N non-zero elements of the first feature map can be mapped to the N cells. so that the second feature information of each cell in the N cells may be determined according to the feature information of the N non-zero elements in the first feature map.

For example, the first feature map is determined as the second feature information of the N cells.

In another example, feature information of non-zero elements is extracted from the first feature map, and the feature information of the non-zero elements is determined as the second feature information of the N cells. For example, for an $i^{th}$ cell in the N cells, the $i^{th}$ cell corresponds to a $j^{th}$ non-zero element in the first feature map. Therefore, feature information of the $j^{th}$ non-zero element is determined as second feature information of the $i^{th}$ cell. For example, as shown in FIG. 7, only N non-zero elements are valid in the generated first feature map with the size of H*W*L. After all zero elements are removed, an N*L-dimensional feature may be finally determined, to form an input representation (Embedding) of a subsequent task for subsequent analysis.

According to the method, after feature extraction is performed on the first image, to determine the second feature information of each cell in the N cells, the following step S404 is performed

S404. Cluster the plurality of cells using the second feature information of the each cell.

In embodiments of the present disclosure, the preset processing performed on the second feature information of each cell in the N cells may be understood as a preset downstream task performed based on the second feature information.

A specific manner of preset processing is not limited in this embodiment of this disclosure.

In some embodiments, the preset processing includes at least one of clustering the N cells, annotating the N cells, and downstream analysis.

In an example, if the preset processing is clustering the N cells, S404 includes the following steps.

S404-A. Clustering the N cells according to the second feature information of each cell in the N cells, to determine M cluster sets of the N cells.

Each cluster set in the M cluster sets includes at least one cell, M being a positive integer less than or equal to N.

It can be learned from the foregoing that in this embodiment of this disclosure, the second feature information fuses the spatial location information and the first feature information, and in response to performing clustering based on the second feature information, clustering accuracy can be improved.

A specifically used clustering algorithm is not limited in this embodiment of this disclosure, for example, may be a general clustering algorithm such as K-means or KNN, or may be a clustering algorithm such as Louvain or Leiden based on community detection.

In some embodiments, this embodiment of this disclosure further includes: selecting P cluster sets from the M cluster sets, P being a positive integer less than or equal to M; and clustering cells in the P cluster sets using the second feature information of the each cell. For example, the cells in the P cluster sets are merged, or the cells in the P cluster sets are classified into more detailed categories, to further implement multi-level and multi-step clustering.

In an example, if the preset processing is to annotate the N cells, in this embodiment of this disclosure, a type annotation model may be provided. The type annotation model includes the submanifold sparse convolution-based autoencoder and a classifier, so that end-to-end type annotation of a cell can be implemented. For example, a probability value that a cell that is determined from another marked data or determined manually may belong to a type may be used as a counterfeiting label, the counterfeiting label is connected after the extracted second feature information through a classifier, and a loss of the classifier is optimized in response to the reconstructed loss being optimized, so that a type annotation model that can directly implement end to end can be finally determined, and performance of the type annotation model is far better than that determined by directly using the counterfeiting label as an annotation result. When clustering has been finished, what to be annotated may be the type of each cell cluster.

In some embodiments, related downstream analysis may be further performed on the cell data according to a result of the clustering or the type annotation. This is not limited in this embodiment of this disclosure.

According to the data processing method provided in this embodiment of this disclosure, cell data is received, and the received cell data is parsed to determine spatial location information and first feature information for each cell of a plurality of cells to which the received cell data corresponds; a first image is generated based on the spatial location information and the first feature information; a first feature map is extracted from the first image, and second feature information of the each cell is determined based on the first feature map. In this way, preset processing (for example, cell clustering or cell annotation) is performed by using the second feature information of the N cells, to determine an accurate processing result. That is, in the embodiments of this disclosure, the to-be-processed data is used to generate the first image, the first image including the spatial location information and the first feature information of each cell in the N cells, then feature extraction is performed on the first image, to determine the second feature information of each cell in the N cells, to encode the spatial location information into a feature, and preset processing such as clustering is directly performed on the second feature information including the spatial location information. The entire data processing process is simple, occupies fewer computing resources, and has high data processing efficiency. In addition, in the embodiments of this disclosure, in response to processing the to-be-processed data, the to-be-processed data is used to generate the first image, and feature extraction is performed on the first image. In response to performing feature extraction on the first image, only the second feature information of each cell in the N cells is extracted. If the first image includes a pixel whose pixel value is zero, feature extraction is not performed on the pixel whose pixel value is zero in the first image, to further save the computing resources and improve the data processing efficiency.

In the embodiments of the present disclosure including the embodiments of both the claims and the specification (hereinafter referred to as "all embodiments of the present disclosure"), the object is preferably configured as a cell, the cell data is preferably configured as spatial transcriptomic data, and the first feature information is preferably configured as gene information.

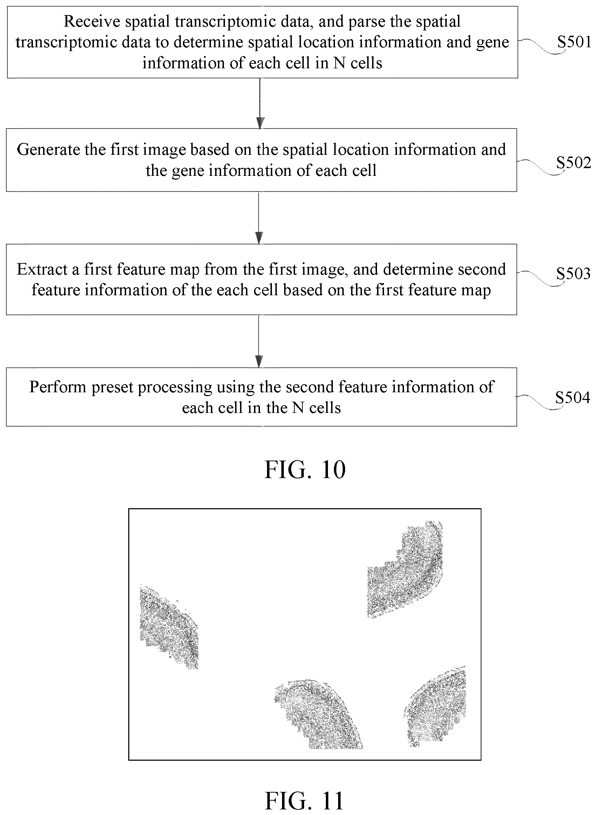

FIG. 10 is a schematic diagram of a processing process of spatial transcriptomic data according to an embodiment of this disclosure. As shown in FIG. 10, this embodiment of this disclosure includes the following steps.

S501. Receive spatial transcriptomic data, and parse the spatial transcriptomic data to determine spatial location information and gene information of each cell in N cells.

In all embodiments of the present disclosure, the spatial transcriptomic data includes the spatial location information and the first feature information of each cell in the N cells. In this case, the spatial location information and the first feature information of each cell in the N cells may be directly read from the spatial transcriptomic data.

In all embodiments of the present disclosure, the spatial transcriptomic data includes gene information captured by a sequencing point. In this case, S501 includes the following steps.

S501-A. Correspond each of the sequencing points to a cell of the N cells, to determine the gene information of the cell, the gene information of the cell including gene data captured by a sequencing point corresponding to the cell.

During actual test, at least one sequencing point may be configured on the cell to capture gene data on the sequencing point, to form spatial transcriptomic data, the spatial transcriptomic data including the gene data captured by the sequencing point. In this embodiment of this disclosure, after the spatial transcriptomic data is determined, the spatial transcriptomic data is parsed to determine the spatial location information and the gene information of each cell in the N cells. Specifically, various sequencing points in the spatial transcriptomic data correspond to the N cells according to a mapping relationship between cells and sequencing points, and gene data captured by the sequencing points corresponding to the cells forms the gene information of the cells, to encapsulate original gene data in a form of a sequencing point into gene information in a form of a cell.

In an example, the spatial transcriptomic data includes the spatial location information of each cell in the N cells, and then the spatial location information of each cell in the N cells may be directly read from the spatial transcriptomic data.

In another example, the spatial transcriptomic data includes spatial location information of each sequencing point, then spatial location information of a sequencing point corresponding to a cell is determined according to a mapping relationship between the sequencing point and the cell, and the spatial location information of a sequencing point is determined as spatial location information of the corresponding cell.

In all embodiments of the present disclosure, the sequencing points in the spatial transcriptomic data correspond to the N cells. Before the gene information of each cell in the N cells is determined, the method further includes: removing an incorrect sequencing point in the spatial transcriptomic data, and then corresponding each of the sequencing points after the incorrect sequencing point is removed in the spatial transcriptomic data to the cell of the N cells, to determine the gene information of each cell in the N cells.

In all embodiments of the present disclosure, the gene information of the N cells generated according to the method is an N*G-dimensional matrix, N being a quantity of cells, G being a quantity of gene types, and an $i^{th}$ row and a $j^{th}$ column of the matrix representing a quantity of genes j expressed by a cell i.

In all embodiments of the present disclosure, the spatial location information of the N cells generated according to the method is an N*2-dimensional matrix, which is recorded as center coordinates of each cell.

S502. Generate the first image based on the spatial location information and the gene information of each cell.

A specific implementation process of S502 is consistent with that of S402. For details, reference may be made to the detailed descriptions of S402.

Specifically, each cell corresponds to a pixel in the first image (in this case, the first image is a blank image or an initial image) based on the spatial location information of each cell and the gene information of each cell is used as color channel information of the pixel corresponding to each cell, so that the spatial transcriptomic data is converted into the first image, the converted first image including the spatial location information and the gene information of each cell during conversion of the spatial transcriptomic data.

In all embodiments of the present disclosure, a blank second image is created; and for each cell in the N cells, a pixel in the blank second image corresponding to the each cell is determined according to the spatial location information of the each cell, and the first image is determined by using the gene information as color channel information of the first image.

A method for creating the blank second image may include: creating the blank second image according to the spatial location information of each cell in the N cells.

For example, a minimum rectangle of the N cells is determined according to the spatial location information of each cell in the N cells. The blank second image is created by using a size of the minimum rectangle as a size of the second image.

In all embodiments of the present disclosure, the first image includes N pixels whose pixel values are not zero and that are in one-to-one correspondence with the N cells, and a quantity of color channels of the first image is a quantity of gene types of the N cells.

It is assumed that the first image is an image of H*W*G, H*W being a size of the first image, and G being a quantity of color channels of the first image, a value of a $g^{th}$ color channel (that is a pixel (h, w, g)) in an $h^{th}$ row and a $w^{th}$ column in the first image represents an amount of a $g^{th}$ gene of a cell whose center coordinates are (h, w).

In response to the generated first image including a pixel whose pixel value is not zero and a pixel whose pixel value is zero, each cell occupies the pixel whose pixel value is not zero, all color channels of the pixel whose pixel value is not zero form gene information of the cell, it indicates that there is no cell at the location, and values of all color channels of the pixel whose pixel value is zero is 0.

Only N pixels in all H*W pixels of the generated first image include information.

In all embodiments of the present disclosure, only information about the pixel whose pixel value is not zero in the first image is extracted. Therefore, in response to the first image being generated, it is not necessary to reduce the pixel whose pixel value is zero included in the first image through a required size of the first image, to compress the resource occupation, but conversion may be directly performed according to the spatial location information of the N cells to determine the first image.

S503. Extract a first feature map from the first image, and determine second feature information of the each cell based on the first feature map.

A specific implementation process of S503 is consistent with that of S403. For details, reference may be made to detailed descriptions of S403.

In all embodiments of the present disclosure, feature extraction is performed on the first image, to determine a first feature map, and second feature information of each cell in the N cells is determined according to feature information of N non-zero elements in the first feature map.

In an example, a size of the first feature map is the same as a size of the first image, a location of a zero element in the first feature map is consistent with a location of a pixel whose pixel value is zero in the first image, and a location of a non-zero element in the first feature map is consistent with a location of a pixel whose pixel value is not zero in the first image.

In all embodiments of the present disclosure, feature extraction is performed on the first image by using a submanifold sparse convolution-based autoencoder, to determine the first feature map.

In all embodiments of the present disclosure, the submanifold sparse convolution-based autoencoder includes at least one submanifold sparse convolution layer.

For a specific implementation of S503, reference is made to the descriptions of S403. Details are not described herein again.

S504. Perform preset processing using the second feature information of each cell in the N cells.

For example, clustering, or annotating, or downstream analysis, or the like is performed on the N cells according to the second feature information of each cell in the N cells.

In all embodiments of the present disclosure, clustering is performed on the N cells according to the second feature information of each cell in the N cells, to determine M cluster sets of the N cells, each cluster set in the M cluster sets including at least one cell, and M being a positive integer less than or equal to N.

In all embodiments of the present disclosure, after the M cluster sets are determined, P cluster sets may be further selected from the M cluster sets, P being a positive integer less than or equal to M; and clustering is performed on cells in the P cluster sets again.

In all embodiments of the present disclosure, this embodiment of this disclosure is applicable to a single-cell analysis platform and is used as a part of an analysis process of the spatial transcriptomic data. A user inputs spatial transcriptomic data (that is, gene information and corresponding spatial locations of a series of cells). The spatial transcriptomic data is first converted into a first image, then second feature information of each cell is extracted by using a submanifold sparse convolution-based autoencoder, and all the cells are divided into a plurality of different clusters by using a clustering algorithm. Clustering may be directly applied to all data or may be applied to partial data according to user selection from a plurality of levels (for example, 1/3 data is first selected and classified into eight classes, and then an eighth class is selected and classified into 10 small classes). Clustering having a low level and a low quantity of clusters may assist in analysis of an anatomical structure, and clustering having a high level and a high quantity of clusters may assist in analysis of a cell or sub-cell structure type. Downstream analysis such as gene spatial differential expression, a Marker gene, and a highly variable gene (HVG) may also be performed according to a result of the clustering.

It can be learned from the foregoing that the data processing method provided in the embodiments of this disclosure can reduce occupation of computing resources and improve data processing efficiency. Therefore, the data processing method provided in this embodiment of this disclosure is applicable to spatial transcriptomic data with an ultra-high resolution and a large field of view. The resolution refers to a sequencing point density, and low-resolution data refers to that the sequencing point density is greater than a cell density. In this case, a sequencing point represents a sum of gene information of a plurality of cells. A high resolution or ultra-high resolution refers to that the sequencing point density is equal to or greater than a cell density. In this case, a single sequencing point represents a cell or sub-cell structure, and one or more sequencing points are merged to form gene information of one cell. The field of view refers to a size of a total space occupied by all cells in a batch of data, the spatial transcriptomic data is usually determined through sequencing on an issue slice, and the large field of view refers to that the slice is relatively large, and generally means that there are a relatively large quantity of cells.

To further describe an effect of the data processing method provided in this embodiment of this disclosure, the beneficial effects of this embodiment of this disclosure are described through experiments.

Table 1 shows GPU view memory occupations of this disclosure and an SEDR technology in different data amounts.

Table 1 Video memory occupations of this disclosure and an SEDR technology

| Method | Data amount (quantity of cells) | GPU video memory occupations |
|---|---|---|
| SEDR | 24510 | 22252 MB |
| This disclosure | 24510 | 1403 MB |
| This disclosure | 73530 | 3073 MB |

It can be learned from Table 1 that the video memory occupation in this disclosure is significantly relatively low and this disclosure is efficiently applicable to the spatial transcriptomic data with the ultra-high resolution and the large field of view.

In addition, in response to cell type guidance being introduced, end-to-end annotation of a cell type can be directly implemented in this disclosure. Generally, there is no relatively reliable verification index for a clustering result, and it is difficult to perform effective quantitative comparison. Therefore, directly comparing an annotation result can more conveniently reflect the superiority of this disclosure. FIG. 11 is a schematic diagram of an annotation result (which corresponds to a single cell as type guidance) on one sample in rat brain primary motor cortex data in multiplexed error-robust fluorescence in situ hybridization (MERFISH) according to this disclosure. Table 2 shows accuracy of cell type annotation results generated by using corresponding single cell data as type guidance on a mouse1_sample1 sample of the data set through this disclosure and other six comparison automatic annotation methods (Seurat, SingleR, scmap, Cell-ID, scNym, and SciBet).

Table 2 Accuracy of this disclosure and comparison methods

| | Thi s disclosur e | Seu rat | Sin gleR | scm ap | Cell -ID | scN ym | Sci Bet |
|---|---|---|---|---|---|---|---|
| Accu racy | 90. 13% | 85. 50% | 87.6 8% | 64. 54% | 16. 45% | 78. 69% | 88. 06% |

It can be learned from Table 2 that annotation accuracy of this disclosure is higher than annotation accuracy of other annotation methods.

In addition, because gene information and spatial information are effectively combined in this disclosure as self-supervision for training, it is also excellent in performance of noisy data. Table 3 shows accuracy of this disclosure and other six comparison methods in response to 30% genes being removed manually on a mouse1_sample1 sample of MERFISH data.

Table 3 Accuracy of this disclosure and comparison methods

|  | Thi s disclosur e | Seu rat | Sin gleR | scm ap | Cell ID | scN ym | Sci Bet |
|---|---|---|---|---|---|---|---|
| Accu racy | 85. 02% | 81. 36% | 80.1 5% | 53. 51% | 15. 41% | 78. 66% | 81. 37% |

It can be learned from Table 3 that annotation accuracy of the method provided in this embodiment of this disclosure is significantly higher than that of other methods after noise is added to data.

According to the data processing method provided in this embodiment, cell data is received, and the received cell data is parsed to determine spatial location information and first feature information for each cell of a plurality of cells to which the received cell data corresponds; a first image is generated based on the spatial location information and the first feature information; a first feature map is extracted from the first image, and second feature information of the each cell is determined based on the first feature map. In this way, preset processing (for example, cell clustering or cell annotation) is performed by using the second feature information of the N cells, to determine an accurate processing result. That is, in the embodiments of this disclosure, the to-be-processed data is used to generate the first image, the first image including the spatial location information and the first feature information of each cell in the N cells, then feature extraction is performed on the first image, to determine the second feature information of each cell in the N cells, to encode the spatial location information into a feature, and preset processing such as clustering is directly performed on the second feature information including the spatial location information. The entire data processing process is simple, occupies fewer computing resources, and has high data processing efficiency. In addition, in the embodiments of this disclosure, in response to processing the to-be-processed data, the to-be-processed data is used to generate the first image, and feature extraction is performed on the first image. In response to performing feature extraction on the first image, only the second feature information of each cell in the N cells is extracted. If the first image includes a pixel whose pixel value is zero, feature extraction is not performed on the pixel whose pixel value is zero in the first image, to further save the computing resources and improve the data processing efficiency.

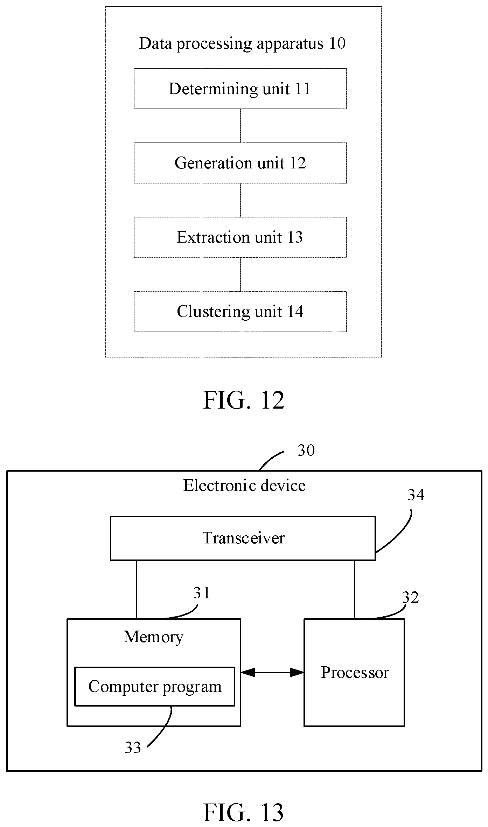

The method embodiments of this disclosure are described in detail above with reference to FIG. 4 to FIG. 11, and apparatus embodiments of this disclosure are described below with reference to FIG. 12 and FIG. 13.

FIG. 12 is a schematic block diagram of a data processing apparatus according to an embodiment of this disclosure. The apparatus 10 may be an electronic device or may be a part of an electronic device.

As shown in FIG. 12, the data processing apparatus 10 includes:

a determining unit 11, configured to receive cell data, and parse the received cell data to determine spatial location information and first feature information for each cell of a plurality of cells to which the received cell data corresponds;

a generation unit 12, configured to generate a first image based on the spatial location information and the first feature information;

an extraction unit 13, configured to extract a first feature map from the first image, and determine second feature information of the each cell based on the first feature map; and

a clustering unit 14, configured to cluster the plurality of cells using the second feature information of the each cell.

In some embodiments, the generation unit 12 is further configured to create a blank second image; and determine, according to the spatial location information of the each cell, a pixel in the blank second image corresponding to the each cell, to obtain the first image, wherein the first feature information is used as color channel information of the pixel.

In some embodiments, the generation unit 12 is further configured to create the blank second image according to the spatial location information of the each cell in the plurality of cells.

In some embodiments, the generation unit 12 is further configured to determine a minimum rectangle enclosing the plurality of cells according to the spatial location information of the each cell; and create the blank second image by using a size of the minimum rectangle as a size of the second image.

In some embodiments, the plurality of cells are N cells, N is a positive integer, the first image includes N pixels whose pixel values are not zero and at least one pixel whose pixel value is zero, the N pixels whose pixel values are not zero being in one-to-one correspondence with the N cells.

In some embodiments, the plurality of cells are N cells, N is a positive integer, the first feature map comprising N non-zero elements in one-to-one correspondence with the N cells, the extraction unit 13 is configured determine the second feature information of the each cell according to feature information of the N non-zero elements in the first feature map.

In some embodiments, a size of the first feature map is the same as a size of the first image, a location of a zero element in the first feature map is consistent with a location of a pixel whose pixel value is zero in the first image, and a location of a non-zero element in the first feature map is consistent with a location of a pixel whose pixel value is not zero in the first image.

In some embodiments, the extraction unit 13 is further configured to perform feature extraction on the first image by using a submanifold sparse convolution-based autoencoder, to determine the first feature map.

In some embodiments, the submanifold sparse convolution-based autoencoder includes at least one submanifold sparse convolution layer.

In some embodiments, the preset processing includes at least one of clustering the N cells, annotating the N cells, and downstream analysis.

In some embodiments, the plurality of cells are N cells, N is a positive integer, the clustering unit 14 is configured to cluster the N cells according to the second feature information of the each cell, to determine M cluster sets, each cluster set in the M cluster sets comprising at least one cell, and M being a positive integer less than or equal to N.

In some embodiments, the clustering unit 14 is further configured to select P cluster sets from the M cluster sets, P being a positive integer less than or equal to M; and cluster cells in the P cluster sets using the second feature information of each cell.

In some embodiments, the plurality of cells are N cells, N is a positive integer, the cell data is spatial transcriptomic data, and the first feature information is gene information, and

the determining unit 11 is further configured to parse the spatial transcriptomic data to determine spatial location information and gene information of the each cell;

the generation unit 12 is further configured to convert the spatial transcriptomic data into the first image based on the spatial location information and the gene information of the each cell.

In some embodiments, the first image includes N pixels whose pixel values are not zero and that are in one-to-one correspondence with the N cells, and a quantity of color channels of the first image is a quantity of gene types of the N cells.

In some embodiments, the spatial transcriptomic data comprises gene data captured by sequencing points, and the determining unit 11 is further configured to correspond each of the sequencing points to a cell of the N cells, to determine the gene information of the cell, the gene information of the cell comprising gene data captured by a sequencing point corresponding to the cell.

In some embodiments, the determining unit 11 is further configured to remove an incorrect sequencing point in the spatial transcriptomic data; and correspond each of the sequencing points after the incorrect sequencing point is removed in the spatial transcriptomic data to the cell of the N cells.

It is to be understood that the apparatus embodiment may correspond to the method embodiment, and for similar description, reference may be made to the method embodiment. To avoid repetition, details are not described herein again. Specifically, the apparatus shown in FIG. 12 may execute the data processing method embodiments, and the foregoing and other operations and/or functions of modules in the apparatus are separately configured for implementing the method embodiments. For brevity, details are not described herein.

The apparatus in this embodiment of this disclosure is described in the foregoing from the perspective of a functional module with reference to the accompanying drawings. It is to be understood that the functional module may be implemented in a form of hardware, or may be implemented through an instruction in a form of software, or may be implemented in a combination of a hardware module and a software module. Specifically, steps in the foregoing method embodiments in the embodiments of this disclosure may be completed by using a hardware integrated logical circuit in the processor, or by using instructions in a form of software. Steps of the methods disclosed with reference to the embodiments of this disclosure may be directly performed and completed by using a hardware decoding processor, or may be performed and completed by using a combination of hardware and a software module in the decoding processor. In some embodiments, the software module may be located in a mature storage medium in the field, such as a random access memory, a flash memory, a read-only memory, a programmable read-only memory, an electrically-erasable programmable memory, or a register. The storage medium is located in the memory. The processor reads information in the memory and completes the steps of the foregoing method embodiments in combination with hardware thereof.

FIG. 13 is a schematic block diagram of an electronic device according to an embodiment of this disclosure. The electronic device is configured to perform the data processing method embodiment.

As shown in FIG. 13, the electronic device 30 may include:

a memory 31 and a processor 32. The memory 31 is configured to store a computer program 33 and transmit the computer program 33 to the processor 32. The processor 32 may invoke the computer program 33 from the memory 31 and run the computer program 33, to implement the method in the embodiments of this disclosure.

For example, the processor 32 may be configured to perform the foregoing method step executed by instructions in the computer program 33.

In some embodiments, the processor 32 may include, but not limited to,
a general-purpose processor, a digital signal processor (DSP), an application-specific integrated circuit (ASIC), a field programmable gate array (FPGA), or another programmable logical device, discrete gate or transistor logical device, or discrete hardware component, and the like

In some embodiments of this disclosure, the memory 31 includes, but not limited to,
a volatile memory and/or a non-volatile memory. The non-volatile memory may be a read-only memory (ROM), a programmable ROM (PROM), an erasable programmable read-only memory (EPROM), an electrically EPROM (EEPROM), or a flash memory. The volatile memory may be a random access memory (RAM), and is used as an external cache. Through exemplary but not limitative description, many forms of RAMs may be used, for example, a static random access memory (SRAM), a dynamic random access memory (DRAM), a synchronous dynamic random access memory (SDRAM), a double data rate synchronous dynamic random access memory (DDR SDRAM), an enhanced synchronous dynamic random access memory (ESDRAM), a synchlink dynamic random access memory (SLDRAM) and a direct rambus random access memory (DR RAM).

In some embodiments of this disclosure, the computer program 33 may be divided into one or more modules. The one or more modules are stored in the memory 31 and executed by the processor 32, to complete the data processing method provided in this disclosure. The one or more modules may be a series of computer program instruction sections that can complete a specific function, and the instruction section is configured for describing an execution process of the computer program 33 in the electronic device.

As shown in FIG. 13, the electronic device 30 may further include:
a transceiver 34, where the transceiver 34 may be connected to the processor 32 or the memory 31.

The processor 32 may control the transceiver 34 to communicate with another device, specifically, may send information or data to the another device, or receive information or data sent by the another device. The transceiver 34 may include a transmitter and a receiver. The transceiver 34 may further include an antenna, and a quantity of the antenna can be one or more.

It is to be understood that various components of the electronic device 30 are connected to each other by a bus system. In addition to including a data bus, the bus system further includes a power bus, a control bus, and a status signal bus.

According to an aspect of this disclosure, a computer storage medium is provided. The computer storage medium stores a computer program. When the computer program is executed by a computer, the computer is enabled to perform the method in the foregoing method embodiments.

An embodiment of this disclosure further provides a computer program product including instructions. When the instructions are executed by a computer, the computer is enabled to perform the method in the foregoing method embodiments.

According to another aspect of this disclosure, a computer program product or a computer program is provided, the computer program product or the computer program including computer instructions, the computer instructions being stored in a computer-readable storage medium. The processor of the computer device reads the computer instructions from the computer-readable storage medium, and the processor executes the computer instructions, to cause the electronic device to perform the method in the method embodiments.

In other words, when software is used to implement embodiments, all or a part of embodiments may be implemented in a form of a computer program product. The computer program product includes one or more computer instructions. When the computer program instructions are loaded and executed on the computer, the procedures or functions according to the embodiments of this disclosure are all or partially generated. The computer may be a general-purpose computer, a dedicated computer, a computer network, or other programmable apparatuses. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line (DSL)) or wireless (for example, infrared, radio, or microwave) manner. The computer-readable storage medium may be any available medium that a computer can access or a data storage device such as a server or a data center that includes one or more integrated available media. The available medium may be a magnetic medium (such as a floppy disk, a hard disk, or a magnetic tape), an optical medium (such as a digital video disc (DVD)), a semiconductor medium (such as a solid state disk (SSD)) or the like.

A person of ordinary skill in the art may be aware that, in combination with the examples described in the embodiments disclosed in this specification, modules and algorithm steps may be implemented by electronic hardware or a combination of computer software and electronic hardware. Whether the functions are performed by hardware or software depends on particular applications and design constraint conditions of the technical solutions.

In the several embodiments provided in this disclosure, it can be understood that the disclosed system, apparatus, and method may be implemented in other manners. For example, the described apparatus embodiment is merely exemplary. For example, the module division is merely logical function division and may be other division in actual implementation. For example, a plurality of modules or components may be combined or integrated into another system, or some features may be ignored or not performed. In addition, the displayed or discussed mutual couplings or direct couplings or communication connections may be implemented by using some interfaces. The indirect couplings or communication connections between the apparatuses or modules may be implemented in electronic, mechanical, or other forms.

The modules described as separate parts may or may not be physically separate, and parts displayed as modules may or may not be physical modules, may be located in one position, or may be distributed on a plurality of network units. Some or all of modules may be selected to achieve the objective of the embodiment solutions according to an actual need. For example, functional modules in the embodiments of this disclosure may be integrated into one processing module, or each of the modules may exist alone physically, or two or more modules are integrated into one module.

**Claims**

1. A data processing method, executable by an electronic device, comprising:

   receiving (S401) cell data, and parsing the received cell data to determine spatial location information and first feature information for each cell of a plurality of cells to which the received cell data corresponds, wherein the received cell data is spatial transcriptomic data, and the plurality of cells are N cells, N is a positive integer;
   generating (S402) a first image based on the spatial location information and the first feature information;
   extracting (S403) a first feature map from the first image by using a submanifold sparse convolution-based autoencoder, the first feature map comprising N non-zero elements in one-to-one correspondence with the N cells, and determining second feature information of the each cell according to feature information of the N non-zero elements in the first feature map; and
   clustering (S404) the plurality of cells using the second feature information of the each cell.

2. The method according to claim 1, wherein the generating a first image based on the spatial location information and the first feature information comprises:

   creating a blank second image; and
   determining, according to the spatial location information of the each cell, a pixel in the blank second image corresponding to the each cell, to obtain the first image, wherein the first feature information is used as color channel information of the pixel.

3. The method according to claim 2, wherein the creating a blank second image comprises:
   creating the blank second image according to the spatial location information of the each cell in the plurality of cells.

4. The method according to claim 3, wherein the creating the blank second image according to the spatial location information of the each cell in the plurality of cells comprises:

   determining a minimum rectangle enclosing the plurality of cells according to the spatial location information of the each cell; and
   creating the blank second image by using a size of the minimum rectangle as a size of the second image.

5. The method according to any one of claims 1 to 4, wherein the plurality of cells are N cells, N is a positive integer, the first image comprises N pixels whose pixel values are not zero and at least one pixel whose pixel value is zero, the N pixels whose pixel values are not zero being in one-to-one correspondence with the N cells.

6. The method according to any one of claims 1 to 5, wherein a size of the first feature map is the same as a size of the first image, a location of a zero element in the first feature map is consistent with a location of a pixel whose pixel value is zero in the first image, and a location of a non-zero element in the first feature map is consistent with a location of a pixel whose pixel value is not zero in the first image.

7. The method according to claim 1, wherein the submanifold sparse convolution-based autoencoder comprises at least one submanifold sparse convolution layer.

8. The method according to any one of claims 1 to 7, wherein the plurality of cells are N cells, N is a positive integer, the clustering the plurality of cells using the second feature information of the each cell comprises:

> clustering the N cells according to the second feature information of the each cell, to determine M cluster sets, each cluster set in the M cluster sets comprising at least one cell, and M being a positive integer less than or equal to N;
> wherein preferably:
> the method further comprises:

>> selecting P cluster sets from the M cluster sets, P being a positive integer less than or equal to M; and
>> clustering cells in the P cluster sets using the second feature information of each cell.

9. The method according to any one of claims 1 to 8, wherein the plurality of cells are N cells, N is a positive integer, and the first feature information is gene information, and
the parsing the received cell data to determine spatial location information and first feature information for each cell of a plurality of cells to which the received cell data corresponds comprises:

> parsing (S501) the spatial transcriptomic data to determine spatial location information and gene information of the each cell;
> the generating a first image based on the spatial location information and the first feature information comprises:

>> converting the spatial transcriptomic data into the first image based on the spatial location information and the gene information of the each cell;
>> wherein preferably:
>> the first image comprises N pixels whose pixel values are not zero and that are in one-to-one correspondence with the N cells, and a quantity of color channels of the first image is a quantity of gene types of the N cells.

10. The method according to claim 9, wherein the spatial transcriptomic data comprises gene data captured by sequencing points, and the parsing the spatial transcriptomic data to determine gene information of the each cell comprises:

> corresponding each of the sequencing points to a cell of the N cells, to determine the gene information of the cell, the gene information of the cell comprising gene data captured by a sequencing point corresponding to the cell;
> wherein preferably:
> before corresponding each of the sequencing points to a cell of the N cells, the method further comprises:

>> removing an incorrect sequencing point in the spatial transcriptomic data; and
>> the corresponding each of the sequencing points to a cell of the N cells comprises:
>> corresponding each of the sequencing points after the incorrect sequencing point is removed in the spatial transcriptomic data to the cell of the N cells.

11. A data processing apparatus (10), comprising:

> a determining unit (11), configured to receive cell data, and parse the received cell data to determine spatial location information and first feature information for each cell of a plurality of cells to which the received cell data corresponds, wherein the received cell data is spatial transcriptomic data, and the plurality of cells are N cells, N is a positive integer;
> a generation unit (12), configured to generate a first image based on the spatial location information and the first feature information;
> an extraction unit (13), configured to extract a first feature map from the first image by using a submanifold sparse convolution-based autoencoder, the first feature map comprising N non-zero elements in one-to-one correspondence with the N cells, and determine second feature information of the each cell based on the first feature map according to feature information of the N non-zero elements in the first feature map; and
> a clustering unit (14), configured to cluster the plurality of cells using the second feature information of the each cell.

12. An electronic device (30), comprising a processor (32) and a memory (31),

the memory (31) being configured to store a computer program (33); and

the processor (32) being configured to execute the computer program (33), to implement the method according to any one of claims 1 to 10.

13. A computer-readable storage medium, configured to store a computer program (33), the computer program (33) causing a computer to perform the method according to any one of claims 1 to 10.

14. A computer program product, comprising computer program instructions, the computer program instructions causing a computer to perform the method according to any one of claims 1 to 10.

**Patentansprüche**

1. Datenverarbeitungsverfahren, das von einer elektronischen Vorrichtung ausführbar ist und Folgendes umfasst:

Empfangen (S401) von Zelldaten und Parsen der empfangenen Zelldaten, um für jede Zelle einer Vielzahl von Zellen, denen die empfangenen Zelldaten entsprechen, räumliche Ortsinformationen und erste Merkmalsinformationen zu bestimmen, wobei die empfangenen Zelldaten räumliche Transkriptomikdaten sind und die Vielzahl von Zellen N Zellen sind, N eine positive Ganzzahl ist;

Erzeugen (S402) eines ersten Bildes auf Basis der räumlichen Ortsinformationen und der ersten Merkmalsinformationen;

Extrahieren (S403) einer ersten Merkmalskarte aus dem ersten Bild unter Verwendung eines Autocodierers, der auf einer Untermannigfaltigkeit einer dünn besetzten Faltung basiert, wobei die erste Merkmalskarte N Nichtnullelemente in einer Eins-zu-eins-Entsprechung mit den N Zellen umfasst, und Bestimmen von zweiten Merkmalsinformationen jeder Zelle gemäß Merkmalsinformationen der N Nichtnullelemente in der ersten Merkmalskarte; und

Clustern (S404) der Vielzahl von Zellen unter Verwendung der zweiten Merkmalsinformationen jeder Zelle.

2. Verfahren nach Anspruch 1, wobei das Erzeugen eines ersten Bildes auf Basis der räumlichen Ortsinformationen und der ersten Merkmalsinformationen Folgendes umfasst:

Erstellen eines leeren zweiten Bildes; und

Bestimmen eines Pixels im leeren zweiten Bild, das jeder Zelle entspricht, gemäß den räumlichen Ortsinformationen jeder Zelle, um das erste Bild zu erhalten, wobei die ersten Merkmalsinformationen als Farbkanalinformationen des Pixels verwendet werden.

3. Verfahren nach Anspruch 2, wobei das Erstellen eines leeren zweiten Bildes Folgendes umfasst:
Erstellen eines leeren zweiten Bildes gemäß den räumlichen Ortsinformationen jeder Zelle in der Vielzahl von Zellen.

4. Verfahren nach Anspruch 3, wobei das Erstellen eines leeren zweiten Bildes gemäß den räumlichen Ortsinformationen jeder Zelle in der Vielzahl von Zellen Folgendes umfasst:

Bestimmen einer minimalen rechteckigen Umschließung der Vielzahl von Zellen gemäß den räumlichen Ortsinformationen jeder Zelle; und

Erstellen des leeren zweiten Bildes unter Verwendung einer Größe des minimalen Rechtecks als einer Größe des zweiten Bildes.

5. Verfahren nach einem der Ansprüche 1 bis 4, wobei die Vielzahl von Zellen N Zellen sind, N eine positive Ganzzahl ist, das erste Bild N Pixel, deren Pixelwerte nicht null sind, und mindestens ein Pixel, dessen Pixelwert null ist, umfasst, wobei die N Pixel, deren Pixelwerte nicht null sind, in einer Eins-zu-eins-Entsprechung mit den N Zellen stehen.

6. Verfahren nach einem der Ansprüche 1 bis 5, wobei eine Größe der ersten Merkmalskarte dieselbe ist wie eine Größe des ersten Bildes, ein Ort eines Nullelements in der ersten Merkmalskarte mit einem Ort eines Pixels konsistent ist, dessen Pixelwert im ersten Bild null ist, und ein Ort eines Nichtnullelements in der ersten Merkmalskarte mit einem Ort eines Pixels konsistent ist, dessen Pixelwert im ersten Bild nicht null ist.

7. Verfahren nach Anspruch 1, wobei der Autocodierer auf Basis einer Untermannigfaltigkeit einer dünn besetzten Faltung mindestens eine dünn besetzte Untermannigfaltigkeitsfaltungsschicht umfasst.

8. Verfahren nach einem der Ansprüche 1 bis 7, wobei die Vielzahl von Zellen N Zellen sind, N eine positive Ganzzahl ist, das Clustern der Vielzahl von Zellen unter Verwendung der zweiten Merkmalsinformationen jeder Zelle Folgendes umfasst:

Clustern der N Zellen gemäß den zweiten Merkmalsinformationen jeder Zelle, um M Clustersätze zu bestimmen, wobei jeder Clustersatz in den M Clustersätzen mindestens eine Zelle umfasst und wobei M eine positive Ganzzahl kleiner als oder gleich N ist;
wobei vorzugsweise:
das Verfahren ferner Folgendes umfasst:

Auswählen von P Clustersätzen aus den M Clustersätzen, wobei P eine positive Ganzzahl kleiner als oder gleich M ist; und
Clustern von Zellen in den P Clustersätzen unter Verwendung der zweiten Merkmalsinformationen jeder Zelle.

9. Verfahren nach einem der Ansprüche 1 bis 8, wobei die Vielzahl von Zellen N Zellen sind, N eine positive Ganzzahl ist und die ersten Merkmalsinformationen Geninformationen sind, und
das Parsen der empfangenen Zelldaten, um räumliche Ortsinformationen und erste Merkmalsinformationen für jede Zelle der Vielzahl von Zellen zu bestimmen, die den empfangenen Zelldaten entsprechen, umfasst Folgendes:

Parsen (S501) der räumlichen Transkriptomikdaten, um räumliche Ortsinformationen und Geninformationen jeder Zelle zu bestimmen;
das Erzeugen eines ersten Bildes auf Basis der räumlichen Ortsinformationen und der ersten Merkmalsinformationen umfasst Folgendes:

Umwandeln der räumlichen Transkriptomikdaten auf Basis der räumlichen Ortsinformationen und der Geninformationen jeder Zelle in das erste Bild;
wobei vorzugsweise:
das erste Bild N Pixel umfasst, deren Pixelwerte nicht null sind und die in einer Eins-zu-eins-Entsprechung mit den N Zellen stehen, und eine Menge von Farbkanälen des ersten Bildes eine Menge von Genarten der N Zellen ist.

10. Verfahren nach Anspruch 9, wobei die räumlichen Transkriptomikdaten Gendaten umfassen, die durch Sequenzierungspunkte erfasst wurden und das Parsen der räumlichen Transkriptomikdaten, um Geninformationen jeder Zelle zu bestimmen, Folgendes umfasst:

Kongruieren von jedem der Sequenzierungspunkte mit einer Zelle der N Zellen, um die Geninformationen der Zelle zu bestimmen, wobei die Geninformationen der Zelle Gendaten umfassen, die von einem Sequenzierungspunkt erfasst wurden, der der Zelle entspricht;
wobei vorzugsweise:
vor dem Kongruieren von jedem der Sequenzierungspunkte mit einer Zelle der N Zellen das Verfahren ferner Folgendes umfasst:

Entfernen eines inkorrekten Sequenzierungspunkts aus den räumlichen Transkriptomikdaten; und
das Kongruieren von jedem der Sequenzierungspunkte mit einer Zelle der N Zellen Folgendes umfasst:
Kongruieren von jedem der Sequenzierungspunkte, nachdem der inkorrekte Sequenzierungspunkt aus den räumlichen Transkriptomikdaten entfernt wurde, mit der Zelle der N Zellen.

11. Datenverarbeitungseinrichtung (10), die Folgendes umfasst:

eine Bestimmungseinheit (11), die dazu ausgelegt ist, Zelldaten zu empfangen und die empfangenen Zelldaten zu parsen, um räumliche Ortsinformationen und erste Merkmalsinformationen für jede Zelle einer Vielzahl von Zellen zu bestimmen, denen die empfangenen Zelldaten entsprechen, wobei die empfangenen Zelldaten räumliche Transkriptomikdaten sind und die Vielzahl von Zellen N Zellen sind, N eine positive Ganzzahl ist;
eine Erzeugungseinheit (12), die dazu ausgelegt ist, ein erstes Bild auf Basis der räumlichen Ortsinformationen und der ersten Merkmalsinformationen zu erzeugen;
eine Extraktionseinheit (13), die dazu ausgelegt ist, unter Verwendung eines Autocodierers, der auf einer Untermannigfaltigkeit einer dünn besetzten Faltung basiert, eine erste Merkmalskarte aus dem ersten Bild zu extrahieren, wobei die erste Merkmalskarte N Nichtnullelemente in einer Eins-zu-eins-Entsprechung mit den N Zellen umfasst, und zweite Merkmalsinformationen jeder Zelle auf Basis der ersten Merkmalskarte gemäß Merkmalsinformationen der N Nichtnullelemente in der ersten Merkmalskarte zu bestimmen; und eine Clustereinheit (14), die dazu ausgelegt ist, die Vielzahl von Zellen unter Verwendung der zweiten Merkmalsinformationen jeder Zelle zu clustern.

12. Elektronische Vorrichtung (30), die einen Prozessor (32) und einen Speicher (31) umfasst,

wobei der Speicher (31) dazu ausgelegt ist, ein Computerprogramm (33) zu speichern; und wobei der Prozessor (32) dazu ausgelegt ist, das Computerprogramm (33) auszuführen, um das Verfahren nach einem der Ansprüche 1 bis 10 zu implementieren.

13. Computerlesbares Speichermedium, das dazu ausgelegt ist, ein Computerprogramm (33) zu speichern, wobei das Computerprogramm (33) einen Computer veranlasst, das Verfahren nach einem der Ansprüche 1 bis 10 durchzuführen.

14. Computerprogrammprodukt, das Computerprogrammanweisungen umfasst, wobei die Computerprogrammanweisungen einen Computer veranlassen, das Verfahren nach einem der Ansprüche 1 bis 10 durchzuführen.

**Revendications**

1. Procédé de traitement de données, exécutable par un dispositif électronique, comprenant :

la réception (S401) de données cellulaires, et l'analyse des données cellulaires reçues pour déterminer des informations d'emplacement spatial et des premières informations caractéristiques pour chaque cellule d'une pluralité de cellules auxquelles correspondent les données cellulaires reçues, dans lequel les données cellulaires reçues sont des données transcriptomiques spatiales, et la pluralité de cellules sont N cellules, N étant un entier positif ;
la génération (S402) d'une première image sur la base des informations d'emplacement spatial et des premières informations caractéristiques ;
l'extraction (S403) d'une première carte de caractéristiques à partir de la première image en utilisant un auto-encodeur basé sur une convolution clairsemée de sous-variété, la première carte de caractéristiques comprenant N éléments non nuls en correspondance biunivoque avec les N cellules, et la détermination de deuxièmes informations caractéristiques de chaque cellule selon les informations caractéristiques des N éléments non nuls dans la première carte de caractéristiques ; et
la mise en grappe (S404) de la pluralité de cellules à l'aide des deuxièmes informations caractéristiques de chaque cellule.

2. Procédé selon la revendication 1, dans lequel la génération d'une première image sur la base des informations d'emplacement spatial et des premières informations caractéristiques comprend :

la création d'une deuxième image vierge ; et
la détermination, selon les informations d'emplacement spatial de chaque cellule, d'un pixel dans la deuxième image vierge correspondant à chaque cellule afin d'obtenir la première image, dans lequel les premières informations caractéristiques sont utilisées comme informations de canal de couleur du pixel.

3. Procédé selon la revendication 2, dans lequel la création d'une deuxième image vierge comprend :
la création de la deuxième image vierge selon les informations d'emplacement spatial de chaque cellule dans la pluralité de cellules.

4. Procédé selon la revendication 3, dans lequel la création de la deuxième image vierge selon les informations d'emplacement spatial de chaque cellule dans la pluralité de cellules comprend :

la détermination d'un rectangle minimal englobant la pluralité de cellules selon les informations d'emplacement spatial de chaque cellule ; et
la création de la deuxième image vierge en utilisant une taille du rectangle minimal comme taille de la deuxième image.

**5.** Procédé selon l'une des revendications 1 à 4, dans lequel la pluralité de cellules sont N cellules, N étant un entier positif, la première image comprend N pixels dont des valeurs de pixel ne sont pas nulles et au moins un pixel dont la valeur de pixel est nulle, les N pixels dont des valeurs de pixel ne sont pas nulles correspondant de manière biunivoque aux N cellules.

**6.** Procédé selon l'une des revendications 1 à 5, dans lequel une taille de la première carte de caractéristiques est la même qu'une taille de la première image, un emplacement d'un élément nul dans la première carte de caractéristiques est cohérent avec un emplacement d'un pixel dont la valeur de pixel est nulle dans la première image, et un emplacement d'un élément non nul dans la première carte de caractéristiques est cohérent avec un emplacement d'un pixel dont la valeur de pixel n'est pas nulle dans la première image.

**7.** Procédé selon la revendication 1, dans lequel l'auto-encodeur basé sur une convolution clairsemée de sous-variété comprend au moins une couche de convolution clairsemée de sous-variété.

**8.** Procédé selon l'une des revendications 1 à 7, dans lequel la pluralité de cellules sont N cellules, N étant un entier positif, la mise en grappe de la pluralité de cellules à l'aide des deuxièmes informations caractéristiques de chaque cellule comprend :

la mise en grappe des N cellules selon les deuxièmes informations caractéristiques de chaque cellule pour déterminer M ensembles de grappes, chaque ensemble de grappes des M ensembles de grappes comprenant au moins une cellule, et M étant un entier positif inférieur ou égal à N ;
dans lequel de préférence :
le procédé comprend en outre :

la sélection de P ensembles de grappes parmi les M ensembles de grappes, P étant un entier positif inférieur ou égal à M ; et
la mise en grappe de cellules dans les P ensembles de grappes à l'aide des deuxièmes informations caractéristiques de chaque cellule.

**9.** Procédé selon l'une des revendications 1 à 8, dans lequel la pluralité de cellules sont N cellules, N étant un entier positif, et les premières informations caractéristiques sont des informations génétiques, et
l'analyse des données cellulaires reçues pour déterminer des informations d'emplacement spatial et des premières informations caractéristiques pour chaque cellule d'une pluralité de cellules auxquelles correspondent les données cellulaires reçues comprend :

l'analyse (S501) des données transcriptomiques spatiales pour déterminer des informations d'emplacement spatial et des informations génétiques de chaque cellule ;
la génération d'une première image sur la base des informations d'emplacement spatial et des premières informations caractéristiques comprend :

la conversion des données transcriptomiques spatiales en la première image sur la base des informations d'emplacement spatial et des informations génétiques de chaque cellule ;
dans lequel de préférence :
la première image comprend N pixels dont des valeurs de pixel ne sont pas nulles et qui correspondent de manière biunivoque aux N cellules, et une quantité de canaux de couleur de la première image est une quantité de types de gènes des N cellules.

**10.** Procédé selon la revendication 9, dans lequel les données transcriptomiques spatiales comprennent des données génétiques capturées par des points de séquençage, et l'analyse des données transcriptomiques spatiales pour déterminer les informations génétiques de chaque cellule comprend :

la mise en correspondance de chacun des points de séquençage à une cellule des N cellules pour déterminer les informations génétiques de la cellule, les informations génétiques de la cellule comprenant des données génétiques capturées par un point de séquençage correspondant à la cellule ;
dans lequel de préférence :
avant de faire correspondre chacun des points de séquençage à une cellule des N cellules, le procédé comprend en outre :

la suppression d'un point de séquençage incorrect dans les données transcriptomiques spatiales ; et
la mise en correspondance de chacun des points de séquençage à une cellule des N cellules comprend :
après la suppression du point de séquençage incorrect dans les données transcriptomiques spatiales, la mise en correspondance de chacun des points de séquençage à la cellule des N cellules.

11. Appareil de traitement de données (10), comprenant :

une unité de détermination (11) configurée pour recevoir des données cellulaires, et analyser des données cellulaires reçues pour déterminer des informations d'emplacement spatial et des premières informations caractéristiques pour chaque cellule d'une pluralité de cellules auxquelles correspondent les données cellulaires reçues, dans lequel les données cellulaires reçues sont des données transcriptomiques spatiales, et la pluralité de cellules sont N cellules, N étant un entier positif ;
une unité de génération (12) configurée pour générer une première image sur la base des informations d'emplacement spatial et des premières informations caractéristiques ;
une unité d'extraction (13) configurée pour extraire une première carte de caractéristiques à partir de la première image en utilisant un auto-encodeur basé sur une convolution clairsemée de sous-variété, la première carte de caractéristiques comprenant N éléments non nuls en correspondance biunivoque avec les N cellules, et déterminer des deuxièmes informations caractéristiques de chaque cellule sur la base de la première carte de caractéristiques selon les informations caractéristiques des N éléments non nuls dans la première carte de caractéristiques ; et
une unité de mise en grappe (14) configurée pour mettre en grappe la pluralité de cellules à l'aide des deuxièmes informations caractéristiques de chaque cellule.

12. Dispositif électronique (30) comprenant un processeur (32) et une mémoire (31), la mémoire (31) étant configurée pour stocker un programme informatique (33) ; et
le processeur (32) étant configuré pour exécuter le programme informatique (33) pour mettre en œuvre le procédé selon l'une des revendications 1 à 10.

13. Support de stockage lisible par ordinateur configuré pour stocker un programme informatique (33), le programme informatique (33) amenant un ordinateur à réaliser le procédé selon l'une des revendications 1 à 10.

14. Produit de programme informatique comprenant des instructions de programme informatique, les instructions de programme informatique amenant un ordinateur à réaliser le procédé selon l'une des revendications 1 à 10.

FIG. 1

Clustering

Space 2

Space 1

FIG. 2

FIG. 3

Receive cell data, and parse the received cell data to determine spatial location information and first feature information for each cell of a plurality of cells to which the received cell data corresponds — S401

Generate a first image based on the spatial location information and the first feature information — S402

Extract a first feature map from the first image, and determine second feature information of the each cell based on the first feature map — S403

Cluster the plurality of cells using the second feature information of the each cell — S404

FIG. 4

First image          First feature map

## FIG. 5

FIG. 6A          FIG. 6B          FIG. 6C          FIG. 6D

## FIG. 7

**FIG. 8**

**FIG. 9**

| Receive spatial transcriptomic data, and parse the spatial transcriptomic data to determine spatial location information and gene information of each cell in N cells | S501 |

| Generate the first image based on the spatial location information and the gene information of each cell | S502 |

| Extract a first feature map from the first image, and determine second feature information of the each cell based on the first feature map | S503 |

| Perform preset processing using the second feature information of each cell in the N cells | S504 |

## FIG. 10

## FIG. 11

Data processing apparatus 10

Determining unit 11

Generation unit 12

Extraction unit 13

Clustering unit 14

FIG. 12

30

Electronic device

34

Transceiver

31

Memory

32

Processor

Computer program

33

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 114496099 A

**Non-patent literature cited in the description**

- **PHAM DUY et al.** stLearn: integrating spatial location, tissue morphology and gene expression to find cell types, cell-cell interactions and spatial trajectories within undissociated tissues. *bioRxiv*, 31 May 2020

- **CHANG YUZHOU et al.** Define and visualize pathological architectures of human tissues from spatially resolved transcriptomics using deep learning. *COMPUTATIONAL AND STRUCTURAL BIOTECHNOLOGY JOURNAL*, 01 January 2022, vol. 20, ISSN 2001-0370, 4600-4617